# EUROPEAN PATENT SPECIFICATION

(11) **EP 3 355 782 B1**
(45) Date of publication and mention of the grant of the patent: **24.01.2024**
(21) Application number: 16852699.4
(22) Date of filing: 30.09.2016
(51) Int. Cl.: A61B 5/06, G06K 7/10, G06K 7/12, G06K 7/14, A61B 34/20

(54) **SIGNAL TAG DETECTION SYSTEMS**
SYSTEME ZUR ERKENNUNG VON SIGNALETIKETTEN
SYSTÈMES DE DÉTECTION DE MARQUEURS À SIGNAUX

(30) Priority: 02.10.2015 US 201562236660 P; 11.01.2016 US 201614992443; 12.08.2016 US 201662374402 P
(43) Date of publication of application: 08.08.2018
(60) Divisional application: 23211243.3
(73) Proprietor: Elucent Medical, Inc., Eden Prairie, MN 55344 (US)
(72) Inventor: VAN DER WEIDE, Daniel W., Brookfield, Wisconsin 53045 (US); VAN DER WEIDE, Noah, Brookfield, Wisconsin 53045 (US); RUDIE, Eric N., Brookfield, Wisconsin 53045 (US); MIEL, David, Broofield, Wisconsin 53045 (US)
(74) Representative: Glawe, Delfs, Moll
(86) International application number: PCT/US2016/054738
(87) International publication number: WO 2017/059228

(56) References cited:
- WO-A1-2015/039039
- WO-A1-2015/063280
- WO-A1-2015/112863
- WO-A2-2014/149183
- DE-A1- 10 112 303
- DE-A1-102011 006 537
- DE-B4-102011 006 574
- US-A- 6 026 818
- US-A1- 2002 107 445
- US-A1- 2003 018 246
- US-A1- 2003 023 161
- US-A1- 2004 123 871
- US-A1- 2007 238 982
- US-A1- 2009 281 419
- US-A1- 2010 305 430
- US-A1- 2014 066 754
- US-A1- 2014 266 175
- US-A1- 2014 309 522
- US-A1- 2016 249 986

## Description

The present application claims priority to U.S. provisional application Serial Number 62/236,660, filed October 2, 2015. The present application claims priority to U.S. Application Serial number 14/992,443, filed January 11, 2016, which claims priority to U.S. Provisional Application Serial Number 62/236,660, filed October 2, 2015. The present application also claims priority to U.S. Provisional Application Serial Number 62/374,402, filed August 12, 2016.

### FIELD

Provided herein are systems for localization of a tag in a tissue of a patient as defined in the claims. Provided herein are systems employing an implantable tag that emits sidebands at defined frequencies upon activation by a magnetic field generated by a remote activating device, and a plurality of witness stations configured to detect such sidebands as defined in the claims. Also provided herein are herein are systems as defined in the claims employing a detection component that is attached to or integrated with a surgical device.

### BACKGROUND

A common and serious challenge for many medical procedures is the accurate localization of treatment areas. For example, the location of lesions, such as tumors that are to undergo treatment, including surgical resection, continues to present a challenge to the medical community. Existing systems are expensive, complex, time-consuming, and often unpleasant for the patient. Such issues are illustrated by the surgical treatment of breast lesions.

A common technique used in breast tumor surgery is wire localization of the lesions. Precise preoperative localization of some breast lesions is necessary before removal of the lesion. Wire localization is used to mark the location of a breast abnormality. The procedure ensures greater accuracy for a breast biopsy or lumpectomy. The surgeon typically uses the wire as a guide to the tissue that needs to be removed. Wire localization is typically conducted in the radiology department of the hospital or surgical center. Mammograms (or in some cases, ultrasound images) are taken to show the location of the breast abnormality. Patients are awake during the placement of the wire, but the breast tissue is numbed to reduce or avoid pain from the needle or the wire. It is possible to feel pressure or pulling sensations during the wire placement. Once images have been taken, and the tissue has been numbed, the radiologist will use a needle to target the breast abnormality. The tip of this needle rests in the location that the surgeon needs to find in order to remove the right tissue. A slender wire is threaded down through the needle and out of its tip, to lodge at the target tissue. The needle is removed, leaving the wire in place. With the wire in place, the patient has another mammogram, to check that the tip of the wire is properly positioned. If the wire is not in the correct place, the radiologist will reposition and re-check it, to ensure accurate placement. When the wire is finally positioned, it will be secured in place with tape or a bandage. The wire localization procedure can take about an hour, and is usually scheduled hours before biopsy or lumpectomy. Thus, the patient must often wait hours for surgery with the wire present in their body and protruding from their skin. The wire is removed, along with some breast tissue, during surgery. This process takes many hours, involves multiple imaging steps, and is inconvenient and unpleasant for the patient-as well as being expensive.

A similar type of procedure is done to localize pulmonary nodules prior to resection. In some cases where pulmonary nodules may be difficult to locate at conventional open surgery or at thoracoscopy, a hook wire, injection of visible dye, or a radionuclide is placed in or around the nodule in an attempt to improve localization prior to removal. This procedure usually takes place in the CT suite prior to the removal of the nodule. The patient is then transported to the surgical unit and the surgeon cuts down on the wire, uses a radionuclide detector, or uses visual landmarks to localize and remove the nodule.

In other types of surgeries and medical procedures, physicians may have trouble locating a target prior to removal or manipulation. Examples of this include the removal of masses, fluid collections, foreign bodies or diseased tissues. Other times, placements of catheters or other percutaneous procedures are performed either without direct visualization or with the lack of a specific guidance modality. Performing procedures without precise guidance can increase the amount of damage to normal tissues and decrease the patient's functional status.

Percutaneous biopsy is a well-accepted, safe procedure performed in virtually every hospital. Biopsy often entails placement of a co-axial guide needle through which the biopsy device is placed into the target. Many of the lesions that are removed, punctured or manipulated as described above have previously undergone successful percutaneous biopsy. The placement of the guide needle for biopsy is an opportunity to place a fiduciary or other localizing system without causing additional tissue trauma than the patient would otherwise undergo.

Many other medical devices and procedures could benefit from improved tissue localization. These include any procedure or test that is degraded by any bodily motion such as cardiac motion, respiratory motion, motion produced by the musculoskeletal system, or gastrointestinal/genitourinary motion. Examples of these include external beam radiation therapy, placement of brachytherapy seeds, imaging tests including but not limited to CT, MRI, fluoroscopy, ultrasound, and nuclear medicine, biopsies performed in any fashion, endoscopy, laparoscopic and thoracoscopic surgery and open surgical procedures.

Improved systems and methods are needed for tissue localization for medical procedures.

US 2003/018246 A1 discloses a system for determining the position of an object inside a body, and specifically relates to the use of such systems in guiding tools used in medical procedures. Said document does not disclose a plurality of witness stations each configured to detect the sideband of the tag or a component that is attachable to a medical device as defined in items c) and d) of claim 1 of the enclosed claim set.

### SUMMARY

The invention is set out in the appended claims. The embodiments of the description which do not fall within the scope of said claims are provided for illustrative purposes only and do not form part of the present invention. Provided herein are systems for localization of one or more tags in a patient (e.g., in a tissue of a patient) as defined in the claims. Provided herein are systems as defined in the claims employing an implantable tag that emits sidebands at defined frequencies upon activation by a magnetic field generated by a remote activating device, and a plurality of witness stations configured to detect such sidebands. Also provided herein are systems as defined in the claims employing a detection component that is attached to or integrated with a surgical device. The methods disclosed herein are not according to the invention as defined in the claims in view of Article 53(c) EPC.

The systems comprise a plurality of components as defined in the claims. A first component comprises one or more tags (which may be used interchangeably with the term "marker") whose location, position, distance, or other properties are to be assessed. The tags can be configured to be positioned in a subject at a surgical location or other clinically relevant location to mark a target region within a body. A second component comprises a remote activating device that generates a magnetic field. The second component may be located in a device positioned near (e.g., below) a subject containing the one or more tags. The third component comprises a plurality of witness stations configured to receive a signal generated by the one or more tags upon being exposed to the magnetic field generated by the second component. The second and third components may be physically contained in the same device. A fourth component comprises a medical device location emitter. The fourth component can be integrated into a medical device or attached or otherwise associated with a medical device. The fourth component comprises one or more emitters (e.g., antennas that emit signals or other types of emitters) that generate signals upon exposure to the magnetic field generated by the second component, said signals detectable by the third component. A fifth component may comprise a computing device comprising a processor that receives information from the witness stations of the third component and generates information about the relative locations, distances, or other characteristics of the tags, the medical device, and the witness stations. The fifth component may comprise a display that displays such generated information to a user of the system.

The first component may be a single tag, or it is two or more tags (e.g., 3, 4, 5, 6, 7, 8, 9, 10, 11, 12, etc.). Where more than one tag is employed, the tags may be of identical type or they are of a different type.

The tag may comprise a ferrite-core coil antenna (e.g., resonant at 100-200 kHz) coupled to an integrated circuit (IC), which is powered by an AC magnetic field at resonance. The core may be contained in an enclosure (e.g., a cylindrical glass or plastic housing). The AC magnetic field originates from the second component. The exciter antenna(s) is/are driven by a conventional oscillator and power amplifier at a level sufficient to power the tag(s). The implanted tag amplitude-modulates (AM's) the continuous wave (CW) carrier power from the exciter, thus emitting sidebands at frequencies defined by a number programmed into the tag's counter. These sidebands, as well as the much stronger CW carrier, may be ultimately detected by the third component.

The tag may comprise a self-resonant object (e.g., a small ferrite core with a wound inductor). The wound inductor possesses inter-winding capacitance that in combination with the inductance produces a high frequency resonant circuit. The tag may comprise a resonant object (e.g., the self-resonant object is equipped with a chip capacitor to produce resonance at a prescribed frequency). The tag may comprise a resonant or self-resonant object with a diode. A diode in combination with an LC circuit produces a sub-harmonic frequency when immersed in a magnetic field of sufficient strength (imposed voltage exceeds the diode's band-gap potential). The tag may comprise a resonant object or self-resonant object with an active modulator (e.g., integrated circuit amplitude modulates resonant circuit). Detection may occur similar to a full duplex (FDX) radio frequency identification (RFID) except that the modulation pattern is a simple sub-harmonic rather than a coded binary pattern; The detection may occur after excitation similar to a half-duplex (HDX) mode of operation.

The tag may be configured for single-use. For example, a tag can be disabled or deactivated (e.g., like an EAS tag). This is particularly useful where multiple tags are used in a procedure where individual tags are turned off to make detection of other tags easier (e.g., to avoid or reduce interference between multiple tags). A burst of energy from an external device may be used to disable or deactivate a tag. The tag may have an internal control component that, upon receiving instruction from an external device, turns the tag on or off (e.g., the tag stops "talking" temporarily or permanently).

The tag may have an exterior length, width, and depth, wherein the length is 30 mm or less (e.g., 20 mm or less, ..., 10 mm or less, ..., 9 mm or less, ..., 8 mm or less, ... , 5 mm or less, . . ., 3 mm or less, ..., etc.), the width is 5 mm or less (e.g., 4 mm or less, ..., 3 mm or less, . . ., 2 mm or less, ..., 1 mm or less, . . . 0.5 mm or less, ..., etc.), and the depth is 5 mm or less (e.g., 4 mm or less, . . ., 3 mm or less, . . ., 2 mm or less, ..., 1 mm or less, .. . 0.5 mm or less, ..., etc.).

The tag may be contained in a housing or no housing is employed. The housing may comprise a biocompatible material. The housing may provide a liquid and/or gas resistant barrier separating the signal source from the exterior of the housing. The housing may be small, permitting administration of the tag through a needle, cannula, endoscope, catheter, or other medical device. The housing may have an exterior length, width, and depth, wherein the length is 30 mm or less (e.g., 20 mm or less, ..., 10 mm or less, . . ., 9 mm or less, ..., 8 mm or less, ... , 5 mm or less, . . ., 3 mm or less, ..., etc.), the width is 5 mm or less (e.g., 4 mm or less, ..., 3 mm or less, ..., 2 mm or less, ..., 1 mm or less, ... 0.5 mm or less, ..., etc.), and the depth is 5 mm or less (e.g., 4 mm or less, . . ., 3 mm or less, . . ., 2 mm or less, . . ., 1 mm or less, ... 0.5 mm or less, ..., etc.). The housing can be of any desired shape. The housing may be cylindrical along the length axis. The housing may be shaped like a grain of rice (e.g., cylindrical with rounded ends). The housing may be shaped like a pillar (e.g., cylindrical with flat ends). The housing may be polygonal along the length axis (e.g., triangular, square, rectangular, trapezoidal, pentagonal, etc., in cross-section). The housing may have struts or other fasteners to keep the tag in place, avoiding migration in tissue. These struts may deploy upon placement in tissue. The fastener may be a biocompatible material that bonds with surrounding tissue.

The housing may be a single uniform component synthesized around the interior components of the tag. The housing may be made of two or more separate segments that are sealed together after introduction of the interior components of the tag. The tag may be completely or partially covered in a coating. The coating may comprise a biocompatible material (e.g., parylene-C, etc.).

The tag may not comprise any power source. For example, the signal is generated from the signal source in response to a magnetic field as the activation event (i.e., electromagnetic induction).

The tag comprises a radio-frequency identification (RFID) chip (e.g., in a housing) as defined in the claims. The RFID chip may comprise a radio-frequency electromagnetic field coil that modulates an external magnetic field to transfer a coded identification number and/or other coded information when queried by a reader device. The RFID chip may collect energy from an EM field generated by the second component (or other device) and then acts as a passive transponder to emit microwaves or UHF radio waves. The RFID chip may be read-only or it is read/write.

The technology is not limited by the nature of the information provided by the RFID chip. The information may include a serial number, lot or batch number, time information (e.g., production date; surgery date; etc.); patient-specific information (e.g., name, family history, drugs taken, allergies, risk factors, procedure type, gender, age, etc.); procedure-specific information; etc. The technology is not limited by the frequency used. The RFID frequency may be in the 120-150 kHz band (e.g., 134 kHz), the 13.56 MHz band, the 433 MHz band, the 865-868 MHz band, the 902-928 MHz band, the 2450-5800 MHz band, or the like. The RFID chip may be incorporated with browser-based software to increase its efficacy. This software may allow for different groups or specific hospital staff, nurses, and patients to see real-time data relevant to the tag, procedure, or personnel. Real-time data may be stored and archived to make use of historical reporting functionality and to prove compliance with various industry regulations. The RFID chip may report sensor data (e.g., temperature, movement, etc.). The RFID chip may contain or collect information that is read at a later time (e.g., after surgery). IInformation may be reviewed during surgery. For example, a message may be provided to the surgeon (e.g., "the chip is just to the left of the tumor") to assist in guiding the surgeon (e.g., optimizing removal of a tumor with the appropriate margins).

The tag may consist of or consists essentially of the signal source and the housing or the signal source, the housing, and the RFID chip. The tag (e.g., via the chip) may emit an ultrasound signal (e.g., gray scale, spectral, or color Doppler) such that the signal is detectable by an ultrasound probe or a hand-held Doppler unit.

A tag may be heated during a procedure (e.g., via exposure to an external energy source). Heating may be used to assist in coagulation or precoagulation of tissue or to provide thermotherapy (see e.g., U.S. Pat. Publ. No. 2008/0213382). Heating may also be used to improve the efficacy of radiation therapy.

The second component may provide a remote activating device having an excitation coil. The excitation coil may be provided in a patch or pad that is placed on the patient or on the operating table, although it can be positioned in any desired location within functional distance of the tags. The remote activating device may provide an AC magnetic field originating from one or more exciter antennas. Where the system is used to locate breast tumors, the patch may encircle the treated breast or is placed otherwise near the breast. Similar approaches may be used for other targeted areas of a body. A pad containing the excitation coil may be placed beneath the patientand a large coil or multiple coils are employed. The excitation coil(s) may comprise or consist of several turns of a flat conductor patterned on a dielectric substrate, or may comprise or consist of magnet wire wound around a suitable mandrel; the coil is powered by an external frequency source, and the magnetic field emanating from the coil penetrates the patient's body to excite the tag, whose emissions are detected by a detection component.

The excitation coil or coils may be contained in a belt that is placed around the subject or a portion of the subject. The external excitation coil may further be used for other aspects of the patient care, such as for radiotherapy or to act as a ground current return pad used in electrosurgery (not being according to the claimed invention as it falls under the exceptions to patentability under Article 53(c) EPC). The remote activating device may emit light (e.g., laser light). The remote activating device may be configured for single use (e.g., is disposable).

The remote activating device may employ an unmodulated constant frequency activation (i.e., the activation signal has constant amplitude and frequency). The remote activating device may employ an unmodulated swept frequency (i.e., the activation signal has constant amplitude and swept frequency between two endpoints). Such devices find use with resonant-type tags such that a detectable change in the activation signal's amplitude occurs when the transmitted frequency coincides with the tag's resonant frequency. The remote activating device may employ a pulsed frequency (i.e., the activation signal comprises brief excitation pulses at a periodic frequency, which may be comprised of two closely-related frequencies whose sum or difference is the response frequency of the tag). The pulsed activation produces a post-pulse sinusoidal decay signal. A tag alters the characteristic of the decaying signal, either in amplitude or time.

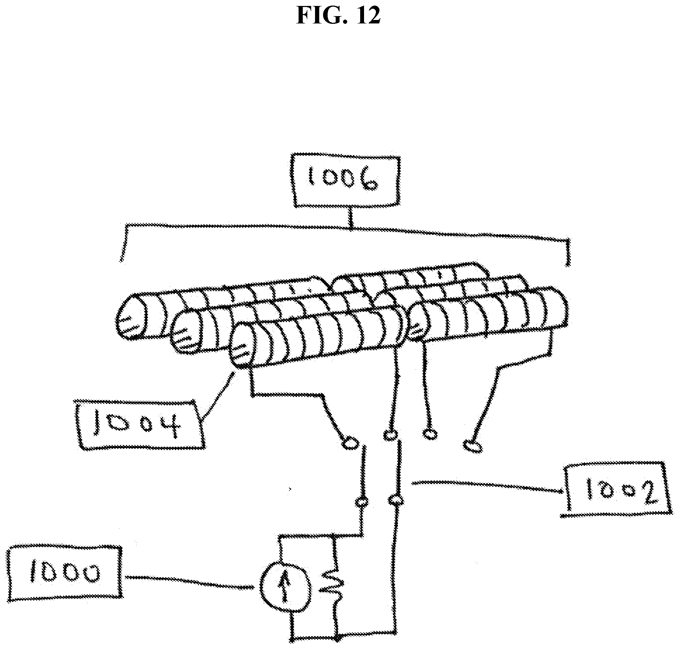

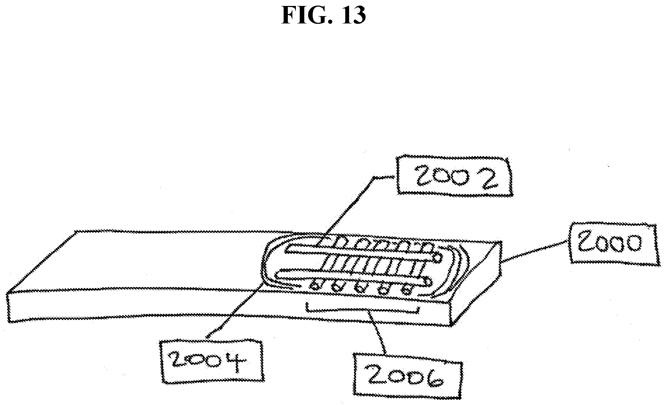

The remote activating device may comprise a hand-held component or a pad or pad-like component. The hand-held component may be lightweight to allow a surgeon to hold and manipulate the component over the course of a procedure (e.g., 5 kg or less, 4 kg or less, 3 kg or less, 2 kg or less, 1 kg or less, 0.5 kg or less, 0.25 kg or less, or any range therein between, e.g., 0.5 to 5 kg, 1 to 4 kg, etc.). The hand-held component may be shaped like a wand, having a proximal end that is held by the physician and a distal end that is pointed towards the treated subject or tissue harboring the tag. The hand-held component may be shaped like an otoscope, having a distal end that terminates at an angle (e.g., right angle) from the body of the component. The remote activating device may comprise a pad or other flat component comprising at least one exciter antenna (e.g., solenoid coil). The remote activating device may comprise an exciter antenna that generates a magnetic field. The remote activating device may have only a single antenna (i.e., is monostatic). The remote activating device may have only two antennas (i.e., is bistatic). The remote activating device may comprise multiple coils (e.g., solenoidal coils) arranged in sub-arrays that are substantially parallel to each other (e.g., as shown in Figure 13). The coils (e.g., solenoidal coils) may be sub-divided into two or more shorter coils sharing the same axis (e.g., as shown in Figure 12).

The magnetic field of the remote activating device may be controlled by a processor running a computer program. The remote activating device comprises a display or user interface that allows the user to control the remote activating device and/or monitor its functions while in use as defined in the claims. The remote activating device may provide a visual, audio, numerical, symbol (e.g., arrows), textual, or other output that assists the user in locating the tag or identifying the distance to or direction of the tag from the remote activating device.

The plurality of witness stations of the third component collectively may provide several antennas at multiple defined locations relative to the tags and configured to receive a signal generated by the one or more tags upon being exposed to the magnetic field generated by the second component.

Each receiving antenna may feed a receiver channel, which is time-division multiplexed (TDM'd) to reduce the receiver complexity. Fixed witness stations of defined locations relative to the tag and each other (e.g., arrayed along the patient) contain one or more (e.g., one to three) witness antennas arranged in a locally orthogonal manner to sense various components of the AC magnetic field from the tag. One or more or all of these witness antennas in the witness stations may be also TDM'd into a receiver channel, reducing complexity, as well as cross-talk between antennas.

Witness antennas may comprise or consist of a ferrite-loaded cylindrical coil antenna, tuned (e.g., with one or more capacitors in parallel) for resonance at the frequency of an exciter (e.g., tag or emitter), (e.g., 100-200 kHz). Typical dimensions of a witness antenna are 3-5 mm diameter and 8-12 mm length, although both smaller and larger dimensions may be employed.

The witness stations may be provided below the patient (e.g., in a pad, garment, or other device positioned below the patient). The witness stations may be integrated into a surgical table or imaging device in which a patient is placed during a medical procedure. The witness stations may be placed on the floor, wall, or ceiling of the operating room or in a medical transport vehicle. The witness stations may be integrated into or attached to a medical device used in the medical procedure.

A fourth component provides a medical device location emitter to allow the system to determine the location, position, distance, or other characteristic of a medical device relative to the tag or tags. The medical device location emitter or emitters may be integrated into a medical device or they are attachable to a medical device. The emitters may be provided in a sleeve that slips over a portion of a medical device. The emitters may operate as and/or comprise the same materials as the tags, but are positioned on or near a medical device rather than within tissue. For example, the emitters may comprise coils that are excited with both carrier and/or sidebands, enabling the emitters to emit signals as though it were a tag.

Location of the emitters may be accomplished geometrically by measuring the quasi-simultaneous power detected from the emitters at a plurality of witness stations (e.g., four or more stations), and using the power differences to perform vector math that determines the location of the emitter without ambiguity. This process is facilitated by a preliminary calibration using a known tag in a known location prior to the procedure.

Vectors describing the location of emitters are used to provide visualization guidance to the surgeon about the spatial relationship of a medical device (e.g., particularly its tip) to an implanted tag, or (e.g., with computational guidance) to a lesion boundary. Use of multiple emitters on a medical device provides vectors to determine the device's principal axis using the same vector math. Where a more complex medical device, such as a robotic surgical system (e.g., da Vinci surgical system) is employed, multiple emitters located on multiple different locations of the device are employed to provide location, orientation, and other position information of multiple components (e.g., arms) of the device. The emitters may be also used as detectors (e.g., provide witness stations on the medical device).

A fifth component may provide one or more computing systems comprising one or more computer processors and appropriate software to analyze, calculate, and display tag and emitter position information. The display may provide a graphical representation of the tag, patient, and/or medical device on a monitor. The display may provide directional information for moving or positioning the medical device. The system may automatically (e.g., robotically) control the medical device or one or more functions thereof. The display may integrate tag and/or medical device information with previously obtained or concurrently obtained medical images of the patient or target tissue (e.g., CT, MRI, ultrasound, or other imaging modalities). For example, an image indicating a tag or tags is fused with an image of the subject's tissue or body region obtained from an imaging device. Information may be analyzed in real-time. Information may be analyzed at one or more discrete time points.

The fifth component may provide command and control functions for a user of the system. The fifth component may have information stored thereon that helps guide the information displayed on the detection component. For example, the information may include data on the type of medical device the detection component is attached to, or what tip or cutting implement is being used with a particular medical device. In this regard, the precise location of the cutting tip of a medical device and its relation to the tag (e.g., distance to the tag) is communicated to the surgeon (e.g., for very precise instructions on cutting tissue). Such information is, for example, manually entered into a control unit or detection component by the user, or automatically found (e.g., by a barcode or other indicator) when a detection component is attached to a particular medical device.

The system finds use with a wide variety of medical devices and procedures. The surgical device may comprise an electrical surgical device that is turned on and off by a user, wherein a control unit that is part of the fifth component allows the remote activating device to generate the magnetic field when the electrical surgical device is off, and prevents the remote activating device from generating the magnetic field when the electrical surgical device is on (e.g., ensuring that the surgical device and detection system do not interfere with one another). The surgical device may comprise a power cord, wherein an AC current clamp is attached to the power cord, wherein the AC current clamp is electrically-linked or wirelessly linked to the control unit, wherein the AC current clamp senses when the electrical surgical device is on or off and reports this to the control unit (e.g., such that the control unit can ensure that the magnetic field from the surgical device and from the remote activating device are not active at the same time).

The surgical device may comprises an electrocautery device, a laser cutting device, a plasma cutting device, or a metal cutting device (e.g., a surgical device manufactured by BOVIE MEDICAL). Additional examples of medical devices that may find use iare found, for example, in the following U.S. Patents: 9,144,453; 9,095,333; 9,060,765; 8,998,899; 8,979,834; 8,802,022; 8,795,272; 8,795,265; 8,728,076; 8,696,663; 8,647,342; 8,628,524; 8,409,190; 8,377,388; 8,226,640; 8,114,181; 8,100,897; 8,057,468; 8,012,154; 7,993,335; 7,871,423; 7,632,270; 6,361,532.

The medical device may have thereon (e.g., provided as part of the fourth component) an indicator for directing the surgeon to the tag or tags. The indicator may provide: i) a spatial orientation indicator (e.g., visual, audible, etc.), and/or ii) a distance-to-tag indicator (e.g., visual, audible, etc.) as defined in the claims. The indicator may comprise a first display for presenting distance to tag information (e.g., visual, audible, lights, color, vibration, tactile, etc.), a second display for presenting vertical axis orientation, such as a preset preferred angle for approaching a tag in a patient (e.g., a visual, audible, lights, colors, vibration, tactile, etc. display); and/or a third display for presenting horizontal orientation (e.g., left to right information so the surgical device can be centered when approaching the tag). The indicator may comprise a plurality of displays (e.g., visual, audible, sensory, etc.) that allow the correct pitch and yaw axes to be employed (to minimize non-target tissue damage), and/or further a display that provides distance to tag information. The medical device may be moved around the patient's body prior to surgery to orient the emitters and the indicator component. A series of lights and/or sounds may be provided on the indicator that guides the surgeon (e.g., the surgeon attempts to keep the lights in a center of an "X" series of lights, and/or to keep the volume of warning sounds off or as low as possible).

The tag is not limited to placement within a particular body region, body part, organ, or tissue. For example, the tag may be placed in the cephalic, cervical, thoracic, abdominal, pelvic, upper extremities, or lower extremities region of the body. The tag may be placed within an organ system, such as the skeletal system, muscular system, cardiovascular system, digestive system, endocrine system, integumentary system, urinary system, lymphatic system, immune system, respiratory system, nervous system or reproductive system. The tag may be placed within an organ. Such organs may include the heart, lungs, blood vessels, ligaments, tendons, salivary glands, esophagus, stomach, liver, gallbladder, pancreas, intestines, rectum, anus, hypothalamus, pituitary gland, pineal gland, thyroid, parathyroids, adrenal glands, skin, hair, fat, nails, kidneys, ureters, bladder, urethra, pharynx, larynx, bronchi, diaphragm, brain, spinal cord, peripheral nervous system, ovaries, fallopian tubes, uterus, vagina, mammary glands, testes, vas deferens, seminal vesicles, and prostate. The tag may be placed within tissues, such as connective, muscle, nervous, and epithelial tissues. Such tissues may include cardiac muscle tissue, skeletal muscle tissue, smooth muscle tissue, loose connective tissue, dense connective tissue, reticular connective tissue, adipose tissue, cartilage, bone, blood, fibrous connective tissue, elastic connective tissue, lymphoid connective tissue, areolar connective tissue, simple squamous epithelium, simple cuboidal epithelium, simple columnar epithelium, stratified epithelium, pseudostratified epithelium, and transitional epithelium.

The tissue region where the tag is located may comprise a lesion. The lesion may be a tumor or a tissue region identified as being at risk for forming a tumor. The lesion may be fibrotic tissue. The lesion may be an inflamed or infected region. The tag may be placed within a lumen to detect function or other process of the organ or provide localizing information. For example, the tag could be swallowed, or placed into a hollow organ via endoscopy. The tissue region may be healthy tissue.

The tag may be placed within a solid tumor. Examples of solid tumors into which the tag may be placed include carcinomas, lymphomas, and sarcomas, including, but not limited to, aberrant basal-cell carcinoma, acinar cell neoplasms, acinic cell carcinoma, adenocarcinoma, adenoid cystic carcinoma, adenoid/pseudoglandular squamous cell carcinoma, adnexal neoplasms, adrenocortical adenoma, adrenocortical carcinoma, apudoma, basal cell carcinoma, basaloid squamous cell carcinoma, carcinoid, cholangiocarcinoma, cicatricial basal-cell carcinoma, clear cell adenocarcinoma, clear cell squamous-cell carcinoma, combined small cell carcinoma, comedocarcinoma, complex epithelial carcinoma, cylindroma, cystadenocarcinoma, cystadenoma, cystic basal-cell carcinoma, cystic neoplasms, ductal carcinoma, endometrioid tumor, epithelial neoplasms, extramammary Paget's disease, familial adenomatous polyposis, fibroepithelioma of Pinkus, gastrinoma, glucagonoma, Grawitz tumor, hepatocellular adenoma, hepatocellular carcinoma, hidrocystoma, Hurthle cell, infiltrative basal-cell carcinoma, insulinoma, intraepidermal squamous cell carcinoma, invasive lobular carcinoma, inverted papilloma, keratoacanthoma, Klatskin tumor, Krukenberg tumor, large cell keratinizing squamous cell carcinoma, large cell nonkeratinizing squamous cell carcinoma, linitis plastica, liposarcoma, lobular carcinoma, lymphoepithelial carcinoma, mammary ductal carcinoma, medullary carcinoma, medullary carcinoma of the breast, medullary thyroid cancer, micronodular basal-cell carcinoma, morpheaform basal-cell carcinoma, morphoeic basal-cell carcinoma, mucinous carcinoma, mucinous cystadenocarcinoma, mucinous cystadenoma, mucoepidermoid carcinoma, multiple endocrine neoplasia, neuroendocrine tumor, nodular basal-cell carcinoma, oncocytoma, osteosarcoma, ovarian serous cystadenoma, Paget's disease of the breast, pancreatic ductal carcinoma, pancreatic serous cystadenoma, papillary carcinoma, papillary hidradenoma, papillary serous cystadenocarcinoma, papillary squamous cell carcinoma, pigmented basal-cell carcinoma, polypoid basal-cell carcinoma, pore-like basal-cell carcinoma, prolactinoma, pseudomyxoma peritonei, renal cell carcinoma, renal oncocytoma, rodent ulcer, serous carcinoma, serous cystadenocarcinoma, signet ring cell carcinoma, signet-ring-cell squamous-cell carcinoma, skin appendage neoplasms, small cell carcinoma, small cell keratinizing squamous cell carcinoma, somatostatinoma, spindle cell squamous cell carcinoma, squamous cell carcinoma, squamous cell lung carcinoma, squamous cell thyroid carcinoma, superficial basal-cell carcinoma, superficial multicentric basal-cell carcinoma, syringocystadenoma papilliferum, syringoma, thymoma, transitional cell carcinoma, verrucous carcinoma, verrucous squamous cell carcinoma, VIPoma, and Warthin's tumor.

Placing the tag may comprise the steps of inserting an introduction device into the subject and introducing the tag through the introduction device into the subject (not being according to the claimed invention in view of the exceptions to patentability under Article 53(c) EPC). The introduction device may be a needle, cannula, or endoscope. The tag may be forced through the introduction device (e.g., via physical force, pressure, or any other suitable technique) and released into the subject at the distal end of the introduction device. After the tag is placed, the introduction device is withdrawn, leaving the tag at the desired location with the subject. The introduction of the tag may be guided by imaging technology.

Multiple tags may be placed into the subject. The tags may be of identical type or may differ (e.g., differ in signal type). The tags may be placed in proximity to one another or at distant locations. Multiple tags may be used to triangulate the location intended for medical intervention.

The tags may be further used as fiducials for radiotherapy (or other targeted therapy). The location of the tags is identified with an external reader and used to place, for example, laser light on the skin surface exactly where the chip is located. This eliminates the need to use X-ray, CT, or fluoroscopy to see the fiducials. This also decreases or eliminates the need to put skin markers (e.g., tattoos) on patients. This also helps in respiratory compensation as the fiducial moves up and down with a tumor in the lung or abdomen. Therefore, one can conduct real-time radiation only when the tumor is in the correct position and decrease damage to the background tissue (e.g., avoid burning a vertical stripe in the patient as the tumor moves up and down). The use as fiducials for director therapy (e.g., radiation therapy) also enhances triangulation as depth information (based on signal strength) assists in localization of the tumor to minimize collateral damage.

Provided herein are systems as defined in the claims and employing one or more or all of: a) a tag (e.g., comprising an antenna; e.g., a coil antenna; e.g., a ferrite-core coil antenna; e.g., that resonates at 100-200 kHz; e.g., coupled to an integrated circuit); b) a remote activation device that generates a magnetic field within a region of the tag; and c) a plurality of witness stations, each of the witness stations comprising an antenna configured to detect information generated by said tag or a change in a magnetic field generated by the remote activation device caused by said tag. The tag emits sidebands at defined frequencies upon activation by a magnetic field and the witness stations detect such sidebands as defined in the claims. The tag may emit the sidebands at frequencies defined by a number programmed into a counter in the tag.

The remote activating device may comprises an excitation coil that is, for example, powered by a generator electrically connected to the remote activating device. The remote activating device may comprise a pad configured to be placed in proximity to (e.g., under, above, beside) a patient having the tag embedded in the patient. The pad may also contain the witness stations.

The witness stations may be tuned to a frequency of the sidebands. Each witness station may comprise a plurality of antennas. Each witness station antenna may feed a receiver channel that is time-division multiplexed. Each antenna of a plurality of antennas within a witness station may be arranged in an orthogonal manner to each other. The witness station antennas may comprise a ferrite-loaded cylindrical coil antenna tuned for resonance at a frequency of the signals to be detected (e.g., from a tag or emitter).

The system may further comprise one or more emitters configured for attachment to a medical device. Any type of attachment may be employed. The one or more emitters may be integrated with the device or may be added to the devices (e.g., via on a sheath that slides over a portion of the device). The emitters may be designed similarly to the tag. For example, the one or more emitters comprise an antenna, wherein the emitters emit sidebands at defined frequencies upon activation by a magnetic field. The one or more emitters may comprise at least two emitters positioned to permit the witness stations to detect orientation of said medical device relative to the tag.

The system may further comprises a computer system that receives information from the plurality of witness stations and generates information about the position of the tag and/or the medical device. The system may further comprises a display that displays the generated information to a user. The display may be on a monitor or on a medical device.

Provided herein are systems as defined in the claims.

The invention as defined in the claims also relates to systems, devices, assemblies, and methods for localization of a tag in a tissue of a patient and which are not according to the claimed invention. For example, provided herein are systems, devices, and methods employing a detection component that is attached to or integrated with a surgical device, where the detection component detects a signal from a tag in a patient, where the tag is activated by remote introduction of a magnetic field. The detection component may comprise three or more sense coils to triangulate a tag location and the distance of the tag from the detection component.

Provided herein and not being according to the claimed invention in view of Article 53(c) EPC are methods for localizing a tissue region of a patient, comprising: a) placing a remote activating device and a patient in proximity to each other, wherein the remote activating device is able to generate a magnetic field, and wherein a tag is located in a tissue of the patient; and b) localizing the tag in the patient by generating a magnetic field with the remote activating device and detecting a signal from the tag using a detector component, wherein the detector component comprises at least one sense coil and is attached to, or integrated with, a surgical device, and wherein the detector component and remote activating device are separate (e.g., not attached to, or part of, each other).

The at least one sense coil may comprise three sense coils that are separated from each other by at least 10 mm (e.g., at least 10 ... 15 ... 20 ... 25 ... 30 ... 40 ... 50 ... 60 ... 70 ... 100 ... 125 ... 150 ... 175 ... or 200 mm). The at least one sense coil may comprise three sense coils arranged in a triangle. The triangle may be an equilateral triangle or approximately an equilateral triangle.

The detector component may comprise a housing with a device-securing component (e.g., an opening in the housing; a snap; tongue and groove; slot; magnetic attachment; etc.). The surgical device may be in inserted through the device-securing opening such that the housing surrounds a portion of the surgical device (and such that said surgical device is secured to said detection component).

The remote activating device, the detector component, and the surgical device may be electrically-linked and/or wirelessly linked to a control unit, wherein the control unit comprises a processor and control software. The detection component may comprise a display (e.g., visual, audible, etc.), and wherein the control unit processes signals from the detection component and provides data that is displayable on the display. The surgical device may comprise an electrical surgical device that is turned on and off by a user, wherein the control unit allows the remote activating device to generate the magnetic field when the electrical surgical device is off, and prevents the remote activating device from generating the magnetic field when the electrical surgical device is on (e.g., ensuring that the surgical device and detection system do not interfere with one another). The surgical device may comprise a power cord, wherein an AC current clamp is attached to the power cord, wherein the AC current clamp is electrically-linked or wirelessly linked to the control unit, wherein the AC current clamp senses when the electrical surgical device is on or off and reports this to the control unit (e.g., such that the control unit can ensure that the magnetic field from the surgical device and from the remote activating device are not active at the same time).

The control, unit and/or detection component, may have information stored thereon that helps guide the information displayed on the detection component. For example, the information may include data on the type of medical device the detection component is attached to, or what tip or cutting implement is being used with a particular medical device. In this regard, the precise location of the cutting tip of a medical device and it's relation to the tag (e.g., distance to the tag) can be communicated to the surgeon (e.g., for very precise instructions on cutting tissue). Such information can, for example, be manually entered into the control unit or detection component by the user, or automatically found (e.g., by a barcode or other indicator) when a detection component is attached to a particular medical device.

The remote activating device may comprise one or more excitation coils. The remote activating device may comprise a pad, and wherein the pad is placed under the patient or under a bed the patient is on. The signal may be an irregularity (e.g., interruption or perturbation) in the magnetic field caused by the tag. The tag may comprise a metal particle (e.g., a ferrite particle).

The detector component may comprise an electronics component, wherein the electronics component comprises a signal processor. The detector component may comprise an electronics component, wherein the electronics component comprises: i) a spatial orientation indicator (e.g., visual, audible, etc.), and/or ii) a distance-to-tag indicator (e.g., visual, audible, etc.). The tissue region may be selected from the group consisting of: a lesion, a tumor, a breast tumor, a blood vessel, a lymph node, and sentinel node.

The detector component may comprise a first display for presenting distance to tag information (e.g., visual, audible, lights, color, vibration, tactile, etc.), a second display for presenting vertical axis orientation, such as a preset preferred angle for approaching a tag in a patient (e.g., a visual, audible, lights, colors, vibration, tactile, etc. display); and/or a third display for presenting horizontal orientation (e.g., left to right information so the surgical device can be centered when approaching the tag). The detector components may comprise a plurality of displays (e.g., visual, audible, sensory, etc.) that allow the correct pitch and yaw axes to be employed (to minimize non-target tissue damage), and/or further a display that provides distance to tag information. The detector component may be moved around the patient's body prior to surgery to orient the detector component. A series of lights and/or sounds may be provided on the detector component that guide the surgeon (e.g., the surgeon attempts to keep the lights in a center of an "X" series of lights, and/or to keep the volume of warning sounds off or as low as possible). For example, the detector component may have an array or geometric shape of lights of different colors that can light up informing the user (e.g., doctor) with regard to location of a tag and/or position of the detector component (and corresponding surgical instrument), such that the user does not have to look away from the surgical field or procedure field.

The signal may comprise: i) a signal detectable by sensory perception; ii) an interruption or perturbation in the magnetic field; or iii) light. The tag comprises a radio-frequency identification (RFID) chip as defined in the claims. The tag may have a length, width, and depth, wherein the length is less than 10 mm, the width is less than 4 mm, and the depth is less than 4 mm. The localizing may comprise detecting a change based on intensity, frequency, color, or sound of the signal. The tag in the tissue of the patient may be detected at a depth of at least 1 mm ... 10 mm ... 45 mm ... 95 mm ... 125 mm ... 174 mm ... or 200 mm.

The methods disclosed herein and not being according to the claimed invention in view of Article 53(c) EPC may further comprise the step of surgically removing a tumor from the patient. Said methods may further comprise the step of administering radiation therapy to the patient using the tag as a fiducial. The patient may comprise a plurality of tags, and wherein the methods further comprise the step of determining locations of the plurality of tags to localize the tissue region in three dimensional space.

The tip of the localizing device (e.g., such as a surgical instrument or an electrocautery system) may be placed in a specific location (e.g., a jig containing a tag at a known distance and orientation from the tip) for calibration. This may require, for example, entering data into the system to describe the length or shape of the instrument.

The invention is defined in the enclosed claims and further relates to systems comprising: a) a detector component comprising a housing and at least one sense coil inside the housing, wherein the detector component detects a signal from a tag inside a patient; and b) a second component selected from the group consisting of: i) a surgical instrument, ii) a remote activating device which generates a magnetic field, iii) a tag that is insertable at a location in a tissue of a patient, and iv) a control unit comprising a processor and a control software, wherein the control unit, when electrically or wirelessly linked to the detector component, provides data to the detector component.

The at least one sense coil may comprise three sense coils that are separated from each other by at least 10 mm (e.g., at least 10 ... 25 ... 45 ... 55 ... 75 ... 137 ... 168 ... or 200 mm). The at least one sense coil may comprise at least three sense coils arranged in a triangle. The triangle may be an equilateral triangle or approximately an equilateral triangle. The housing may have a device-securing component (e.g., opening in housing; slot; snap; etc.). The second component may comprise the surgical device, and wherein the surgical device is in inserted through the device-securing opening such that the housing surrounds a portion of the surgical device and the surgical device is secured to the housing. The surgical instrument may comprises hand-held surgical instrument.

The tag generates the signal when exposed to the magnetic field. The signal may be an irregularity in the magnetic field. The detection component may further comprises a display (e.g., visual, audible, tactile, etc.), and wherein the control unit processes signals from the detection component and provides the data that is displayable on the visual display. The data may comprise distance to tag data and/or orientation data. The control unit, when electrically or wirelessly linked to the detector component and the remote activating component, may cause the remote activating device to generate the magnetic field when the electrical surgical device is off, and may prevent the remote activating device from generating the magnetic field when the electrical surgical device is on. The surgical device may comprise a power cord, wherein an AC current clamp is attached to the power cord.

The surgical device may comprise an electrocautery device, a laser cutting device, a plasma cutting device, or a metal cutting device. The remote activating device may comprise an excitation coil. The remote activating device may comprise a pad or other generally flat component.

The tag may comprise a metal particle (e.g., ferrite particle). The detector component may comprise an electronics component, wherein the electronics component comprises a signal processor. The detector component may comprise an electronics component, wherein the electronics component comprises: i) a visual spatial orientation indicator, and/or ii) a distance-to-tag indicator. The tag may have a length, width, and depth, wherein the length is less than 10 mm, the width is less than 4 mm, and the depth is less than 4 mm.

Provided herein are detector components comprising: i) a housing having a device-securing component (e.g., opening in housing; snap; slot; etc.); and ii) at least one sense coil inside the housing, wherein the detector component detects a signal from a tag inside a patient. IThe at least one sense coil may comprise three sense coils that are separated from each other by at least 10 mm (e.g., 10 mm ... 50 .. 200 mm). The at least one sense coil may comprise at least three sense coils (e.g., arranged in a triangle, or otherwise able to triangulate the position and distance of tag). The triangle may be an equilateral triangle or approximately an equilateral triangle. The detector component may further comprise an electronics component, wherein the electronics component comprises a signal processor. The detector component may further comprise an electronics component, wherein the electronics component comprises: i) a spatial orientation indicator, and/or ii) a distance-to-tag indicator.

The invention is defined in the claims and further relates to detector components comprising: i) a housing; and ii) at least three sense coils inside the housing (e.g., arranged in a triangle or otherwise able to triangulate the position and distance of the tag) wherein the detector component is able to detect a signal from a tag inside a patient. The three sense coils may be separated from each other by at least 10 mm (e.g., 10 mm ... 100 mm ... 200 mm). The triangle may be an equilateral triangle or approximately an equilateral triangle. The detector component may further comprise an electronics component, wherein the electronics component comprises a signal processor. The detector component may further comprises an electronics component, wherein the electronics component comprises: i) a spatial orientation indicator, and/or ii) a distance-to-tag indicator. The housing may have a device-securing opening therein.

The invention is defined in the claims and further relates to devices comprising: a) a hand-held surgical instrument, and b) a detector component attached to, or integrated with, the hand-held surgical instrument, wherein the detector component comprises a housing and at least one sense coil inside the housing, wherein the detector component is able to detect an irregularity in a magnetic field.

The at least one sense coil may comprise three sense coils that are separated from each other by at least 10mm ... 30mm ... 50mm ... or 200 mm. The at least one sense coil may comprise three sense coils arranged in a triangle or other arrangement able to triangulate the location of a tag in a patient. The triangle may be an equilateral triangle or approximately an equilateral triangle. The detector component may comprise a housing with a device-securing component (e.g., opening therein, snap, slot, or other connector) and wherein the surgical device is attached to the detection component via the device-securing component (e.g., surgical device is in inserted through the opening such that the housing surrounds a portion of the surgical device). The hand-held surgical instrument may comprise an electrocautery device, a laser cutting device, a plasma cutting device, or a metal cutting device. The hand-held surgical instrument may comprise a power cord, and wherein an AC current clamp is attached to the power cord. The detector component may comprise an electronics component, wherein the electronics component comprises a signal processor. The detector component may comprise an electronics component, wherein the electronics component comprises: i) a spatial orientation indicator (e.g., display), and/or ii) a distance-to-tag indicator.

The tag may comprise a self-resonant object (e.g., a small ferrite core with a wound inductor). The wound inductor possesses inter-winding capacitance that in combination with the inductance produces a high frequency resonant circuit. The tag may comprise a resonant object (e.g., self-resonant object is equipped with a chip capacitor to produce resonance at a prescribed frequency). The tag may comprise a resonant or self-resonant object with a diode. A diode in combination with LC circuit produces a sub-harmonic frequency when immersed in a magnetic field of sufficient strength (imposed voltage exceeds the diode's band-gap potential). The tag may comprise a resonant object or self-resonant object with an active modulator (e.g., integrated circuit amplitude modulates resonant circuit). Detection occurs similar to a full duplex (FDX) radio frequency identification (RFID) except that the modulation pattern is a simple sub-harmonic rather than a coded binary pattern.

A magnetic field is provided by a remote activating device within the region of the tag, as defined in the claims. The remote activating device may cause the activation event when in proximity (e.g., within a meter, ... 0.5 meters, ... 0.3 meters, ... 0.2 meters, ... 0.1 meters, ... 0.05 meters, ..., etc.) to the tag. The intensity of the signal may increase with closer proximity of the activating device and the tag. The tag may not comprise any energy storage devices (e.g., battery, capacitor, etc.).

The remote activating device may employ an unmodulated constant frequency activation (i.e., the activation signal has constant amplitude and frequency). The tag may produce an irregularity in the activation field. The sensing method detects a shift in either amplitude or frequency induced by the tag's presence.
The remote activating device may employ an unmodulated swept frequency (i.e., the activation signal has constant amplitude and swept frequency between two endpoints). Such devices find use with resonant-type tags such that a detectable change in the activation signal's amplitude occurs when the transmitted frequency coincides with the tag's resonant frequency.

The detection component may comprise a series of lights (LEDs) (e.g., 5 lights) which are lit to indicate proximity, distance, or direction to the tag. The user may have control over the strength of the magnetic field produced by the remote activating device. Internal algorithms embodied in the software may control the magnetic field. The user may select one or more algorithms from a menu. Algorithms may reduce or increase the sensitivity of the remote activating device based on its distance from the tag. The display on the detection component may display numerals (e.g., numbers on an LCD screen for reporting distance).

An image from an imaging component may be associated with data collected by the detection component. A user display may provide an image of the tissue from the subject (e.g., obtained from MRI, CT, ultrasound, or other imaging modality) and overlays information about the location of the tag, the detection component, and/or a surgical tool used by the surgeon.

The remote activating device may comprise an excitation coil. The excitation coil may be provided in a patch or pad that is placed on the patient or on the operating table. Where the system is used to locate breast tumors, the patch may encircle the treated breast or may be placed otherwise near the breast. A pad containing the excitation coil may be placed beneath the patient. A large coil or multiple coils may be employed. The excitation coil(s) may comprise or consist of several turns of a flat conductor patterned on a dielectric substrate, or may comprise or consist of magnet wire wound around a suitable mandrel; the coil is powered by an external frequency source, and the magnetic field emanating from the coil penetrates the patient's body to excite the tag, whose emissions (in some embodiments at a higher harmonic of the excitation or in some temporal or spectral combination unique to the tag) are detected by the detection component.

The detection component may be attached to or integrated with a surgical device, such as an electrosurgical device (e.g., electrocautery device such as a BOVIE device), cutting device, ablation device, or the like. A single housing may contain all components of the detection component and the surgical device. Alternatively, a bracket or other component is used to connect a component of a detection component to a surgical device. A holder may be used to mount both the electrosurgical device and the detection component together. The detection component or a component thereof may be attached to or integrated into another type of medical device that is used in the desired surgical procedure (e.g., clamps, endoscopes, bronchoscopes, extended bronchoscopes, dissection tools, lasers, laparoscopes, thoracoscopes, etc.).

The invention is defined in the claims and further relates to systems comprising the above tags, remote activating devices, and detection component. Said systems may comprise the tag and detection component. Systems may further comprise other hardware (e.g., RFID reader), software, instructions, medical devices (e.g., cutting tools, imaging devices, tissue ablation devices, syringes, introduction needles/cannulas/endoscopes, sterilization components, etc.), pharmaceuticals, or other components useful, necessary, or sufficient for conducting a procedure with the tag. The system may comprise a computer that provides command and control functions for the tag and/or detection component. The software may collect and analyze procedure data, information from an RFID chip, or other information generated during a procedure using the tag. A computer may comprise a display for displaying information to the treating physician, radiologist, patient, or other personnel involved in a procedure.

A fiduciary or localizing system may be placed during an endoscopic procedure. For example, during colonoscopy, gastroscopy, duodenoscopy, cystoscopy, etc., a fiducial could be attached to a polyp or other mass. This fiducial is then localized during a subsequent procedure such as, for example, a laparoscopic colon resection or other procedure.

The tag may comprise a fixing component on the outer surface (e.g., of the housing, if present) to anchor the tag in the desired location. The fixing component may be a hook, barb, or other physical extension. The fixing component may be deployable upon placement. The fixing component may be a textured surface. The fixing component may be an adhesive.

It will be appreciated that the systems and methods described herein may be applied to other uses not comprised by the present invention as defined in the claims, including non-medical uses. The technology finds use in any situation where localization of a tag is desired, including, but not limited to, surgical procedures, diagnostic procedures, veterinary procedures, food analysis, industrial applications, and environmental applications.

### DEFINITIONS

As used herein, the terms "processor" and "central processing unit" or "CPU" are used interchangeably and refer to a device that is able to read a program from a computer memory (e.g., ROM or other computer memory) and perform a set of steps according to the program.

As used herein, the terms "computer memory" and "computer memory device" refer to any storage media readable by a computer processor. Examples of computer memory include, but are not limited to, RAM, ROM, computer chips, digital video discs (DVD), compact discs (CDs), hard disk drives (HDD), optical discs, and magnetic tape. The computer memory and computer processor may be part of a non-transitory computer (e.g., in the control unit). Non-transitory computer readable media may be employed, where non-transitory computer-readable media comprises all computer-readable media with the sole exception being a transitory, propagating signal.

As used herein, the term "computer readable medium" refers to any device or system for storing and providing information (e.g., data and instructions) to a computer processor. Examples of computer readable media include, but are not limited to, DVDs, CDs, hard disk drives, magnetic tape and servers for streaming media over networks, whether local or distant (e.g., cloud-based).

As used herein, the term "in electronic communication" refers to electrical devices (e.g., computers, processors, etc.) that are configured to communicate with one another through direct or indirect signaling. For example, a conference bridge that is connected to a processor through a cable or wire, such that information can pass between the conference bridge and the processor, are in electronic communication with one another. Likewise, a computer configured to transmit (e.g., through cables, wires, infrared signals, telephone lines, airwaves, etc.) information to another computer or device, is in electronic communication with the other computer or device.

As used herein, the term "transmitting" refers to the movement of information (e.g., data) from one location to another (e.g., from one device to another) using any suitable means.

As used herein, the term "subject" or "patient" refers to any animal (e.g., a mammal), including, but not limited to, humans, non-human primates, companion animals, livestock, equines, rodents, and the like, which is to be the recipient of a particular treatment. Typically, the terms "subject" and "patient" are used interchangeably herein in reference to a human subj ect.

As used herein, the term "subject/patient suspected of having cancer" refers to a subject that presents one or more symptoms indicative of a cancer (e.g., a noticeable lump or mass) or is being screened for a cancer (e.g., during a routine physical). A subject suspected of having cancer may also have one or more risk factors. A subject suspected of having cancer has generally not been tested for cancer. However, a "subject suspected of having cancer" encompasses an individual who has received an initial diagnosis (e.g., a CT scan showing a mass) but for whom the stage of cancer is not known. The term further includes people who once had cancer (e.g., an individual in remission).

As used herein, the term "biopsy tissue" refers to a sample of tissue (e.g., breast tissue) that is removed from a subject for the purpose of determining if the sample contains cancerous tissue. Biopsy tissue may be obtained because a subject is suspected of having cancer. The biopsy tissue is then examined (e.g., by microscopy; by molecular testing) for the presence or absence of cancer.

As used herein, the term "sample" is used in its broadest sense. In one sense, it is meant to include a specimen or culture obtained from any source, as well as biological and environmental samples. Biological samples may be obtained from animals (including humans) and encompass fluids, solids, tissues, and gases. Biological samples may include tissue, blood products, such as plasma, serum and the like.

### DESCRIPTION OF DRAWINGS

FIG. 1 shows an exemplary 3-dimenstional positioning of tags, witness stations, and locator emitters on a wand configured to fit over a medical device.
FIG. 2 shows an exemplary pad configuration with multiple witness stations.
FIG. 3 shows an exemplary witness station configuration having three orthogonal coils arranged to minimize cross-talk.
FIG. 4 shows an exemplary positioning of tags, a pad comprising a remote activating device and/or witness stations, and a medical device.
FIG. 5A shows an exemplary power supply connected to coils positioned within a remote activating device.
FIG. 5B shows a control unit (60) that is attached to both a remote activating device (50) and a detection component-medical device assembly (21). The remote activating device (50) has an excitation coil (55). The detection component - surgical device assembly (21) is attached to the control unit (60) via connection wire (65).
Figure 6 shows an exemplary detection component (20) having a detection component housing (25) which contains three sense coils (30) and an electronics component (35). The housing (25) also has a device-securing opening (40) therein.
Figure 7A shows an exemplary medical device (electrocautery device) from U.S. Pat. 8,998,899. Said device is not according to the invention and is present for illustrative purposes only.
Figure 7B shows an exemplary device/assembly of the present disclosure, showing a detection component attached to the medical device of Figure 7A.
Figure 8 shows an exemplary detection component - medical device assembly (21), wherein the surgical device is inserted through the device-securing opening of the detection component housing.
Figure 9 shown a photograph of an exemplary detection component-medical device assembly.
Figure 10 shows a photograph of an exemplary detection component with a visual display (45) located therein.
Figure 11 shows a photograph of a side view of an exemplary detection component-medical device assembly.
Figure 12 shows an exemplary arrangement of one layer or sub-array of coils.
Figure 13 shows an exemplary array of substantially orthogonal coils on or in a patient bed.

### DETAILED DESCRIPTION

The invention is set out in the appended claims. The embodiments of the description which do not fall within the scope of said claims are provided for illustrative purposes only and do not form part of the present invention. Provided herein are systems for localization of one or more tags in a patient (e.g., in a tissue of a patient) as defined in the claims. Provided herein are systems as defined in the claims employing an implantable tag that emits sidebands at defined frequencies upon activation by a magnetic field generated by a remote activating device, and a plurality of witness stations configured to detect such sidebands. Also provided herein are systems as defined in the claims employing a detection component that is attached to or integrated with a surgical device.

The invention is defined in the claims and further relates to systems, devices, and methods (not being according to the claimed invention) employing one or more or all of a) one or more tags placed into an object, such as a patient; b) a remote activating device that generates an electromagnetic field within a region of the one or more tags; c) a plurality of witness stations that receive information from the one or more tags that have been exposed to the electromagnetic field; d) one or more emitters positioned on a medical device that are exposed to the electromagnetic field and that emit information received by the witness stations; and e) a computer system for analyzing information received by the witness station and generating and displaying information about the positions of the medical device and/or tag or tags (e.g., relative location, relative distance, orientation, etc.).

The systemsas defined in the claims may be used in any context where the position of a tag is desired and/or where the relative position of another device (e.g., a medical device) is relative to a tag or tags. While the specification focuses on medical uses in human tissues, it should be understood that the systems as defined in the claims may find broader use, including non-human uses (e.g., use with non-human animals such as livestock, companion animals, wild animals, or any veterinary settings). For example, the system may be used in environmental settings, agricultural settings, industrial settings, or the like.

The tag may comprise a coil antenna. The coil antenna may be a ferrite-core coil antenna. The coil antenna may resonate at 100-200 kHz. The coil antenna may be coupled to an integrated circuit (IC). The IC may be powered by an AC magnetic field at resonance (e.g., provided by an activating device). The coil antenna may be provided in an enclosure (e.g., a glass or plastic enclosure). The tag (with enclosure, if present) may have an exterior length, width, and depth, wherein the length is 30 mm or less (e.g., 20 mm or less, ..., 10 mm or less, ..., 9 mm or less, ..., 8 mm or less, . . ., 5 mm or less, ..., 3 mm or less, ..., etc.), the width is 5 mm or less (e.g., 4 mm or less, . . ., 3 mm or less, . . ., 2 mm or less, ..., 1 mm or less, ... 0.5 mm or less, ..., etc.), and the depth is 5 mm or less (e.g., 4 mm or less, .. ., 3 mm or less, ..., 2 mm or less, ..., 1 mm or less, ... 0.5 mm or less, ..., etc.). The tag, with enclosure, may be shaped as an approximately 2x4 mm cylinder or smaller.

The tag amplitude-modulates (AM's) the continuous wave (CW) carrier power from the magnetic field from the activating device, thus emitting sidebands at frequencies defined by a number programmed into the tag's counter. These sidebands, as well as the much stronger CW carrier if desired, are detected for the purpose of analyzing the position of the tag. The use of side bands permits a corresponding detector or detectors (e.g., witness stations) to detect the specific signal from the tag (e.g., using a lock-in amplifier tuned to the side band), without detecting background noise. This allows for precise, real-time detection and analysis of one or more tags, including analysis of relative position and distance from another object (e.g., a medical device).

The remote activating device may comprise a lock-in amplifier or device similar to a lock-in amplifier. The lock-in type amplifier may be configured for narrow-band detection to provide an omnidirectional detection system for determining location of the tag. A modulating signal may be employed and a higher harmonic caused by nonlinearity by the tag is detected. For example, a 40 kHz signal may be provided and the system looks for a 2nd harmonic of 80 kHz generated when the tag is present. The nonlinearity may be a semiconductor diode junction excited by current flowing through a coil of the tag.

As an alternative to use of a lock-in type amplifier, cavity ringdown may be employed. Temporal pulses are emitted and the detector looks for resonator decay over time. Not only is the phase and frequency of the ringdown signal specific to the particular tuning of the tag, but also the exponential decay (to a given threshold) of the ringdown signal's envelope is a relative indicator of the tag's distance from the excitation source.

Any number of other tag designs may be employed. The tag may comprise or consist of a ferrous pellet or particle. When the ferrous object is introduced within a magnetic field, the object creates an irregularity in the alternating magnetic field which is detectable by sense coils contained within witness stations, producing a phase and amplitude shift from null. The null is restored when the ferrous object is physically equidistant to two sense coils.

The tag may comprise a self-resonant object (e.g., a small ferrite core with a wound inductor). The wound inductor possesses inter-winding capacitance that in combination with the inductance produces a high frequency resonant circuit. Detection occurs, for example, using the approach described above for the ferrous pellet or, for example, using a Grid Dip Oscillator (GDO). The GDO has a resonant circuit that radiates an electromagnetic field. When proximal to the self-resonant object of the same frequency, power transfer from the GDO to the self-resonant object induces a detectable change in the GDO power. The tag may comprise a resonant object (e.g., self-resonant object is equipped with a chip capacitor to produce resonance at a prescribed frequency). The tag may comprise a resonant or self-resonant object with a diode. A diode in combination with LC circuit produces a sub-harmonic frequency when immersed in a magnetic field of sufficient strength (imposed voltage exceeds the diode's band-gap potential). The tag may comprise a resonant object or self-resonant object with an active modulator (e.g., integrated circuit amplitude modulates resonant circuit). Detection occurs similar to a full duplex (FDX) radio frequency identification (RFID) except that the modulation pattern is a simple sub-harmonic rather than a coded binary pattern.

The tag comprises a radio-frequency identification (RFID) chip (e.g., in a housing) as defined in the claims. The RFID chip may comprise a radio-frequency electromagnetic field coil that modulates an external magnetic field to transfer a coded identification number and/or other coded information when queried by a reader device. The RFID chip may collect energy from an EM field generated by the activating device (or other device) and then acts as a passive transponder to emit microwaves or UHF radio waves. A reader (which can be part of the activation device or another device) may send a signal to the RFID chip and reads its response. The RFID chip may be read-only or it is read/write.

The technology is not limited by the nature of the information provided by the RFID chip. The information may include a serial number, lot or batch number, time information (e.g., production date; surgery date; etc.); patient-specific information (e.g., name, family history, drugs taken, allergies, risk factors, procedure type, gender, age, etc.); procedure-specific information; etc. The technology is not limited by the frequency used. The RFID frequency may be in the 120-150 kHz band (e.g., 134 kHz), the 13.56 MHz band, the 433 MHz band, the 865-868 MHz band, the 902-928 MHz band, the 2450-5800 MHz band, or the like. The RFID chip may be incorporated with browser-based software to increase its efficacy. This software may allow for different groups or specific hospital staff, nurses, and patients to see real-time data relevant to the tag, procedure, or personnel. Real-time data may be stored and archived to make use of historical reporting functionality and to prove compliance with various industry regulations. The RFID chip may report sensor data (e.g., temperature, movement, etc.). The RFID chip may contain or collect information that is read at a later time (e.g., after surgery). Information may be reviewed during surgery. For example, a message may be provided to the surgeon (e.g., "the chip is just to the left of the tumor") to assist in guiding the surgeon (e.g., optimizing removal of a tumor with the appropriate margins).

The tag may consist of or consists essentially of the signal source and the housing or the signal source, the housing, and the RFID chip. The tag (e.g., via the chip) may emit an ultrasound signal (e.g., gray scale, spectral, or color Doppler) such that the signal is detectable by an ultrasound probe or a hand-held Doppler unit.

The tag may be configured for single-use. A tag can be disabled or deactivated (e.g., like an EAS tag). This is particularly useful where multiple tags are used in a procedure where individual tags are turned off to make detection of other tags easier (e.g., to avoid or reduce interference between multiple tags). A burst of energy from an external device may be used to disable or deactivate a tag. The tag may have an internal control component that, upon receiving instruction from an external device, turns the tag on or off (e.g., the tag stops "talking" temporarily or permanently).

The localization tag may be contained in a housing or no housing is employed. The housing may comprise a biocompatible material. The housing may provide a liquid and/or gas resistant barrier separating the signal source from the exterior of the housing. The housing may be small, permitting administration of the tag through a needle, cannula, endoscope, catheter, or other medical device. The housing may have an exterior length, width, and depth, wherein the length is 30 mm or less (e.g., 20 mm or less, ..., 10 mm or less, . . ., 9 mm or less, ..., 8 mm or less, . . ., 5 mm or less, . . ., 3 mm or less, . . ., etc.), the width is 5 mm or less (e.g., 4 mm or less, . . ., 3 mm or less, . . ., 2 mm or less, ..., 1 mm or less, . . . 0.5 mm or less, ..., etc.), and the depth is 5 mm or less (e.g., 4 mm or less, ..., 3 mm or less, . . ., 2 mm or less, ..., 1 mm or less, . . . 0.5 mm or less, ..., etc.). The housing can be of any desired shape. The housing may be cylindrical along the length axis. The housing may be shaped like a grain of rice (e.g., cylindrical with rounded ends). The housing may be shaped like a pillar (e.g., cylindrical with flat ends). The housing may be polygonal along the length axis (e.g., triangular, square, rectangular, trapezoidal, pentagonal, etc., in cross-section). The housing may have struts or other fasteners to keep the device in place, avoiding migration in tissue. These struts may deploy upon placement in tissue. The fastener may be a biocompatible material that bonds with surrounding tissue. The tag may comprise an anti-migration surface. The anti-migration surface may be textured to reduce movement of the tag when in contact with tissue or a target location. The anti-migration feature may be made of any desired material, including, but not limited to titanium, nitinol, polyethylene, terepthalate, nylon, polyethylene, polytetrafluoroethylene, polypropylene, polyurethane, polyamide, silicone, and combinations thereof.

The housing may be a single uniform component synthesized around the interior components of the tag. The housing may be made of two or more separate segments that are sealed together after introduction of the interior components of the tag. The tag may be completely or partially covered in a coating. The coating may comprise a biocompatible material (e.g., parylene-C, etc.). The tag may not comprise any power source. For example, the signal may be generated from the signal source in response to a magnetic field as the activation event (i.e., electromagnetic induction).

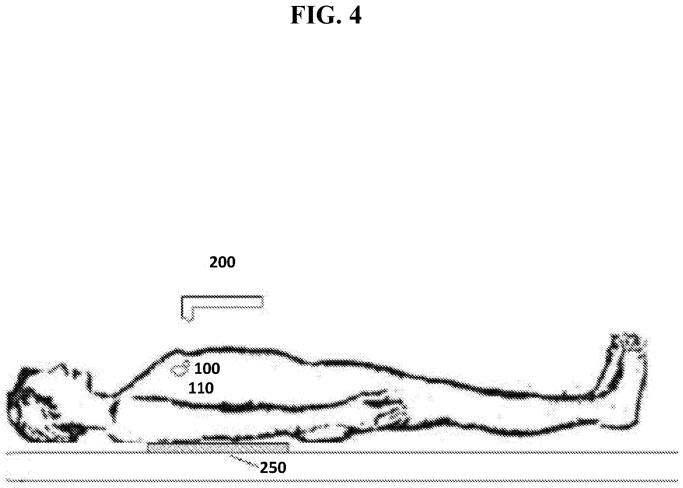

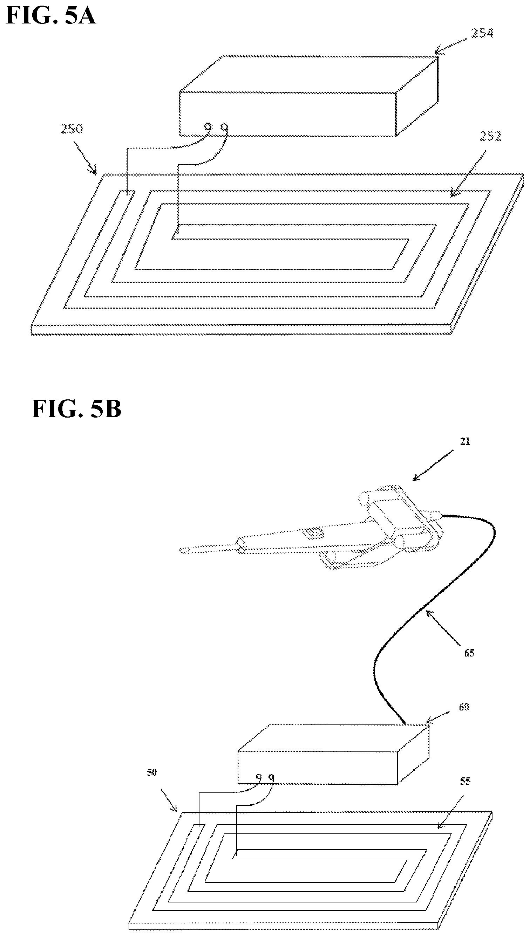

The remote activating device may comprise one or more excitation coils contained in a flat pad. The pad may be sized and shaped to fit beneath a patient during a medical procedure. The pad may be integrated or placed on a surgical table or imaging system, may be integrated into the patient's clothing, or otherwise placed in the surgical field. FIG. 5A provides exemplary remote activating device **250** containing an excitation coil **252** and connected to a generator **254** by wires. FIG. 4 shows an exemplary placement of the remote activating device **250** between a surgical table and a subject, the subject having a tissue mass (e.g., tumor) **110** and a tag **100** inserted near the tissue mass **110.** A medical device **200** is positioned above the patient. The tag **100** and the medical device **200** are within range of a magnetic field that is generated by the remote activating device **250.** The excitation source of the activation device may be a synthesized and stabilized frequency source (e.g., oscillator) whose output is gain-controlled (e.g., via an intermediate amplifier) and provided to a power amplifier to maintain adequate power levels for driving the implanted tag or emitter on a medical device.

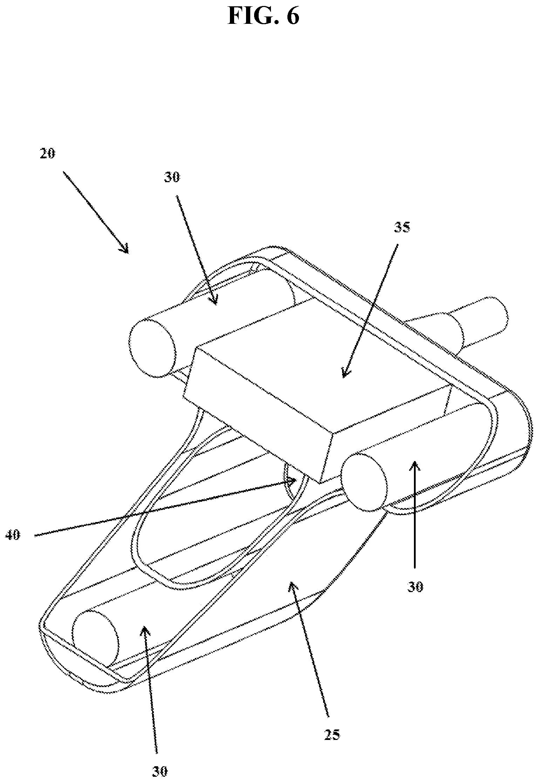

The witness stations may be also included in the same device (e.g., pad) as the remote activating device or they provided in a different device. Witness stations may be provided on or associated with a medical device. For example, FIG. 6 shows a component **20** configured to fit around a medical device that comprises a housing **25** that contains three witness stations **30** that are arrange in a triangle configuration and an electronics component **35** for receiving and processing signals received by the witness stations. The housing **25** has a device-securing opening **40** therein, that allows a medical device to be inserted and secured in place.

Each witness antenna may comprise or consist of a ferrite-loaded cylindrical coil antenna, tuned (e.g., with one or more capacitors in parallel) for resonance at the frequency of an exciter (e.g., tag or emitter) (e.g., typically 100-200 kHz). Typical dimensions of a witness antenna are 3-5 mm diameter and 8-12 mm length, although both smaller and larger antenna may be employed. Witness station antenna may have a ferrite core size of 0.25 x 1 inch and contains 75-80 turns of a 10/46 (10 strands of #46) Litz wire which provides 0.157mH (Q=53) (75 Turns).

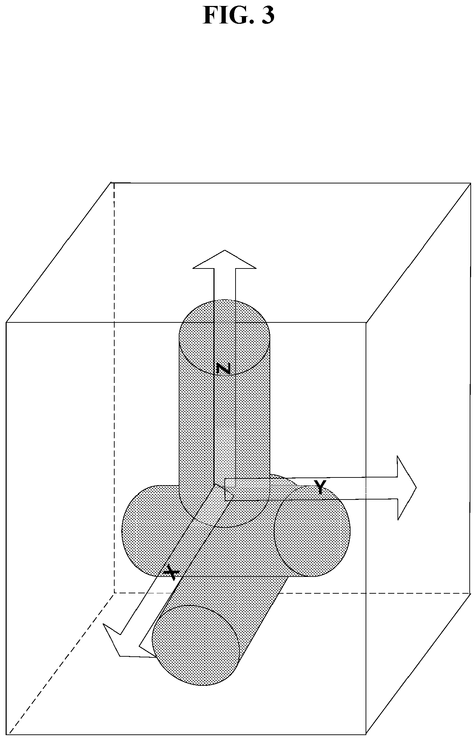

Each witness station may contain 1-3 witness antennas oriented orthogonally to each other and further arranged to have minimum cross-talk (i.e., interference with one another). FIG. 3 shows an exemplary configuration with three antennas, one oriented in the x plane, one in the y plane, and one in the z plane.

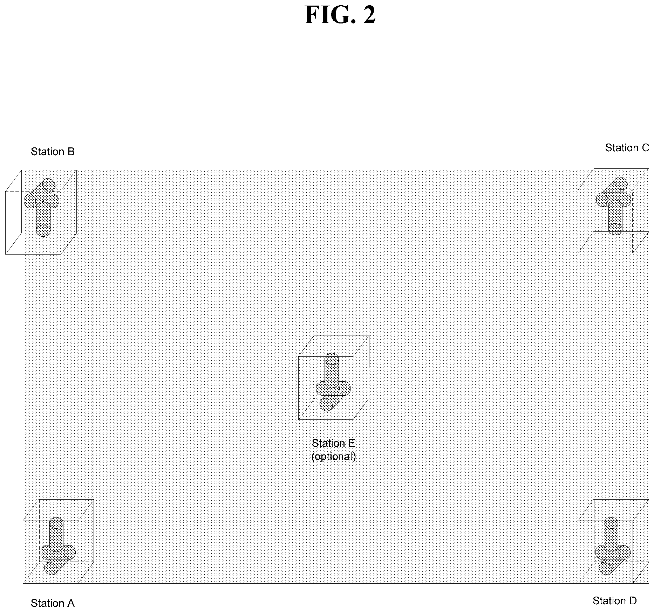

FIG. 2 shows an exemplary arrangement of witness stations within a flat pad with four witness stations positioned at each of the four corners (labeled Station A, Station B, Station C, and Station D) and a fifth optional station (Station E) positioned in the center. Any number of stations may be employed (e.g., 1, 2, 3, 4, 5, 6, 7, 8, 9, 10, 11, 12, etc.) in any desired position and orientation.

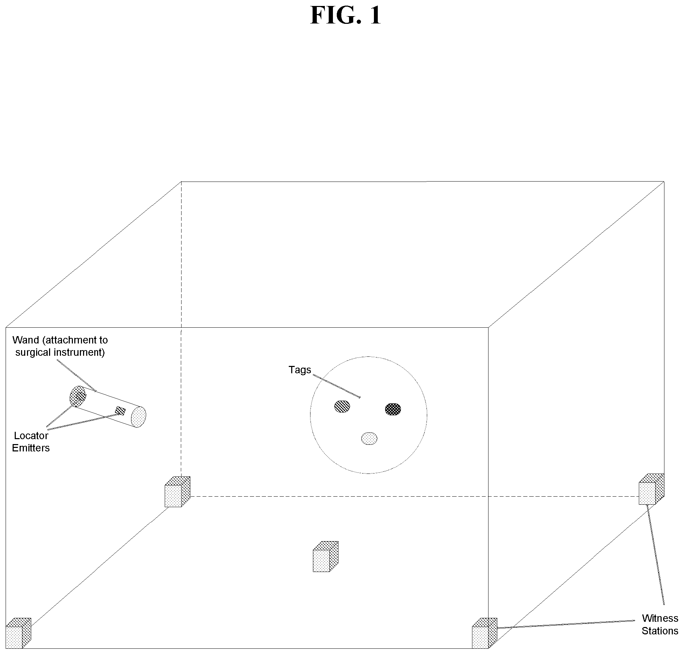

FIG. 1 shows an exemplary configuration of a witness station configuration as shown in FIG. 2 in three-dimensional space relative to three tags located in an object above the witness station and relative to a wand (e.g., attached to a surgical instrument) having two locator emitters.

The component housing the witness stations further comprises one or more receiver channels for collecting information obtained by the antennas of the witness stations. The receiver may comprise or consists of one or more channels, each channel fed by one or more (via a multiplexing switch) witness antennas.

Location of an implanted tag or an emitter on a medical device may be accomplished geometrically by measuring the quasi-simultaneous power detected from these tags at multiple (e.g., four or more) witness stations, and using the power differences to perform vector math that determines the location of the tag without ambiguity. This process may be facilitated by a preliminary calibration using a known tag in a known location prior to the procedure.

Vectors describing the location of implanted tags or medical device emitters may be used to provide visualization guidance to the surgeon about the spatial relationship of the medical device (particularly its tip) to the implanted tag, or (with computational guidance) to a lesion boundary. Multiple emitters on the medical device further provide vectors to determine the medical device's principal axis using the same vector math.

The component comprising the witness stations may comprise an analog front-end. For example, the analog input to the receiver may comprise or consists of a current-to-voltage (transimpedance instrumentation) preamplifier (http://www followed by analog.com/en/products/amplifiers/instrumentation-amplifiers/ad8421.html#product-overview) whose output drives a synchronous detector (http://www. Followed by analog.com/en/products/rf-microwave/iq-modulators-demodulators/iq-demodulators/ad630.html#product-overview) that takes the unknown signal from the witness antennas and compares it to the known CW exciter signal, effectively filtering out the strong exciter signal and providing the amplitude modulation frequency of the tag as its output. This function is similar to that of a lock-in amplifier, where the (unmodulated) frequency reference is used to place a narrow-band notch filter onto the reference, recovering a much smaller modulation in the presence of noise.

Subsequent stages in the analog front-end may provide additional bandpass and lowpass filtering and gain. For example, the output of these stages may be provided to a precision rectifier to directly determine a DC voltage proportional to the received signal strength from the instant antenna, or the unrectified signal is digitized using conventional D/A techniques.

A digital back-end of the receiver may accept as input either a digital version of the DC voltage level or may first perform a digital demodulation of the AC signal. Either approach results in a numerical indication of the signal strength due to the instant antenna. This signal varies with distance d between the instant witness antenna and the tag according to an inverse integer power relationship, e.g. 1/d⁶. Detailed considerations of the variation of signal strength with distance are found in http://robotics. followed by eecs.berkeley.edu/~pister/290Q/Papers/Antennas%20propagation%20interference/near%20fi eld%20path%20loss.pdf, which is a paper entitled "A Near Field Propagation Law & A Novel Fundamental Limit to Antenna Gain Versus Size," submitted to IEEE APS Conference July 2005," author is Dr. Hans Schantz.

Consulting the above reference and inverting the experimentally-determined near-field signal strength versus distance relationship (e.g. 1/d⁶) enables the magnitude of a given distance vector to be determined with accuracy. Accumulating signal strengths and corresponding (post-calibration) distances from all active channels, an acceptably self-consistent solution to tag location in a given grid relative to the witness antennas is determined. One witness antenna can be designated as the origin of a world coordinate system, and all subsequent distances determined from that point. This can be done for both implanted tag signals and signals from the emitters associated with a surgical tool.

Location data for the tag as well as for the emitters may be used to provide indications to the surgeon of tag-medical device distance. This information may be presented in relative format, e.g. one or more visual indicators of tag direction relative to the tip of the medical device. It can also be more quantitative, e.g. a number of bars or lights corresponding to the number of centimeters between the tag and the medical device tip. Further use of the distance data may be employed for rendering a simple image of the medical device and its relative orientation and distance to the tag.

The emitters associated with a medical device may comprise any feature that creates a detectable signal in a magnetic field. The emitter may be of the nature of any of the tags described herein. Coils mounted onto a surgical tool or otherwise used for calibration may be driven directly with a modulated version of the exciter signal from the activation device so that these coils serve as substitute tags and can be located by the receiver of the witness stations component in the same manner as an implanted tag. The modulation to drive these coils can be accomplished with a conventional switch or frequency mixer as modulator, or by numerical means via a digital synthesizer.

The component that contains the emitters may further comprise a display to assist the user in directing the medical device to the tag during a surgical procedure. A visual or audio display may be provided on or associated with the medical device that receives location information about the tag from the computer system. The display may be one or more directional indicators such as LEDs, that indicate direction and/or distance to the tag. Color changes may be employed to indicate "on target" versus "off target" positions. The display may comprise a first display for presenting distance to tag information (e.g., visual, audible, lights, color, vibration, tactile, etc.); a second display for presenting vertical axis orientation, such as a preset preferred angle for approaching a tag in a patient (e.g., a visual, audible, lights, colors, vibration, tactile, etc. display); and/or a third display for presenting horizontal orientation (e.g., left to right information so the surgical device can be centered when approaching the tag). The display may comprise a plurality of displays (e.g., visual, audible, sensory, etc.) that allow the correct pitch and yaw axes to be employed (to minimize non-target tissue damage), and/or further a display that provides distance to tag information. A series of lights and/or sounds may be provided on the display that guide the surgeon (e.g., the surgeon attempts to keep the lights in a center of an "X" series of lights, and/or to keep the volume of warning sounds off or as low as possible).

An LED may be employed for the display, and the LED is configured to emit light without the use of a power source (e.g., battery) within the tag. The two terminals (anode and cathode) of an LED may be in contact with a coiled wire that, when in proximity to a changing magnetic field, induces a voltage and current into the coil, lighting the LED. The technology is not limited by the nature of the LED used. The LED may emit light in the visual spectrum. The LED may emit light in the ultraviolet or infrared spectrums. When a LED is switched on, electrons recombine with holes within the device, releasing energy in the form of photons. This effect is called electroluminescence and the color of the light (corresponding to the energy of the photon) is determined by the energy band gap of the semiconductor. The LED may comprise a lens or case (e.g., epoxy lens or case) surrounding a leadframe comprising an anvil and post. The anvil may comprise a semiconductor die in a reflective cavity and is connected to the post with a wire bond. The LED may be configured to produce different color effects (e.g., red, white, blue) (e.g., with Y3A15O12:Ce phosphor coating for white LED). The semiconductor material may be one or more of gallium arsenide, aluminum gallium arsenide, gallium arsenide phosphide, aluminum gallium indium phosphide, gallium (III) phosphide, indium gallium nitride, zinc selenide, silicon carbide (as a substrate), silicon (as a substrate), diamond, boron nitride, aluminum nitride, and aluminum gallium indium nitride. The LED may be a quantum dot LED. The LED may be a single-die LED to minimize its size profile.

There is no limitation by the mode of tag placement and a wide variety of placements techniques are contemplated, even though said methods are not according to the present invention as defined in the claims in view of Article 53(c) EPC, including, but not limited to, open surgery, laparoscopy, endoscopy, via endovascular catheter, etc. The tags may be placed by any suitable device, including, but not limited to, syringes, endoscopes, bronchoscopes, extended bronchoscopes, laparoscopes, thoracoscopes, etc. An exemplary protocol is provided below.

A patient previously identified as having a breast tumor is admitted to a medical facility. The patient is initially sent to radiology. The radiologist examines prior imaging information identifying the target tumor. The subject is administered a local anesthetic, usually lidocaine or a derivative, using a needle introduced percutaneously. The subject is positioned in an imaging device, generally either ultrasound, conventional mammography, or a stereotactic unit. The location of the tumor is determined. An introducer needle (usually 6-20 gauge corresponding to 4.1-0.81 mm) is inserted either into or just proximal to the tumor and a biopsy needle is placed through the introducer needle and a specimen is obtained using a variety of methods (suction, mechanical cutting, freezing to fix the position of the tissue followed by mechanical cutting). After the specimen is obtained and sent for pathologic examination, a 6-20 gauge (4.1-0.81 mm) tag delivery needle is inserted into the coaxial introducer needle to the tissue with the distal open end positioned at the lesion. A tag is inserted into the proximal end of the delivery needle and delivered by plunger through the opening at the distal end of the needle and into the tissue. Likewise, the tag could have been pre-positioned at the distal end of the delivery needle. Proper location of the tag is confirmed via imaging. The delivery needle is withdrawn, leaving the tag in place in the breast tissue.

This type of procedure can be performed in an analogous manner in virtually any body space, organ, or pathologic tissue with the intent of localizing that tissue or space for further diagnosis or treatment of any kind. Areas of particular interest include but are not limited to the following organs, and disease processes that take place within them: brain, skull, head and neck, thoracic cavity, lungs, heart, blood vessels, gastrointestinal structures, liver, spleen, pancreas, kidneys, retroperitoneum, lymph nodes, pelvis, bladder, genitourinary system, uterus, ovaries, and nerves.

During surgery not being according to the claimed invention in view of Article 53(c) EPC), the patient may placed onto an operating table with the surgical area exposed and sterilized. The surgeon is provided with the imaging information showing the location of the target tissue (e.g., tumor) and tag. An incision is made at the location of the entry point of the placement needle. The remote activating device is placed in proximity to the tissue to activate the tag. The detection component comprising the witness stations detects a signal from the tag and allows the surgeon to guide the direction medical device toward the tumor. Once the tumor is localized, the surgeon removes the appropriate tissue and, optionally, removes the tag.

The system finds use in surgery with the tags placed as fiducials on or in the body. The relative position of the tags and any surgical instruments is located using the electromagnetic field. This information is communicated to a physician in real-time using a variety of methods including by not limited to visual (computer screens, direction and depth indicators using a variety of methods, haptic feedback, audio feedback, holograms, etc.), and the position of the instruments displayed on any medical images such as CT, MRI, or PET scans in 2D or 3D. This data finds use to guide the physician during a procedure, or is used as a training method so that physicians can perform a virtual procedure. Such system may be integrated into or provide alternative approaches to existing surgical systems, such as the STEALTH system (Medtronic) for applications such as neurosurgeries.

The invention is defined in the claims and further relates to systems, devices, assemblies, and methods for localization a tag in a tissue of a patient. For example, provided herein are systemsas defined in the claims employing a detection component that is attached to or integrated with a surgical device, where the detection component can detect a signal from a tag in a patient, where the tag is activated by remote introduction of a magnetic field. The detection component may comprise three sense coils arranged in a triangle.

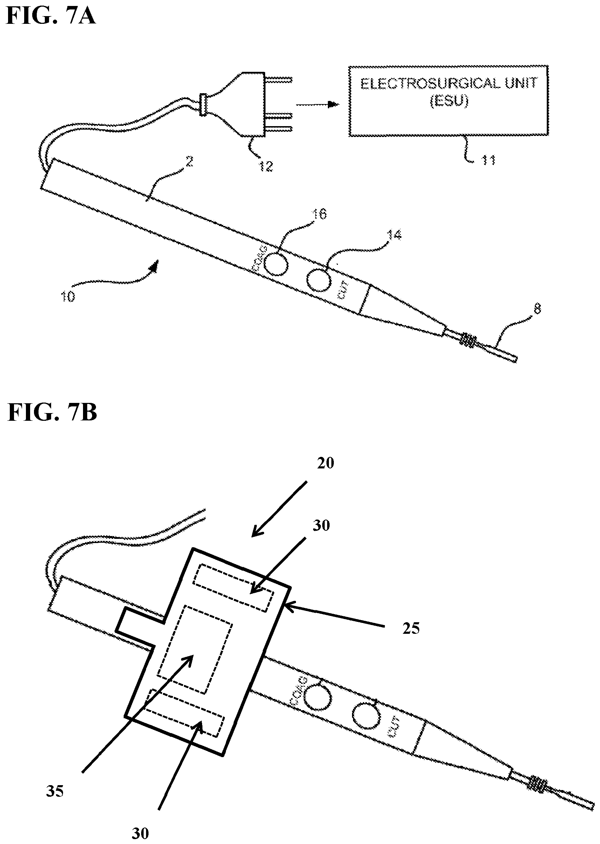

Figure 7A shows an exemplary medical device (electrocautery device) from U.S. Pat. 8,998,899. Specifically, figure 7B shows a surgical instrument (10), with a housing (2) having a coagulation button (16) and a cut mode button (14). The tip of the surgical instrument (10) is attached to electrode (8), that may be used for cutting and/or cauterizing tissue. The surgical device (10) is attached to an electrical surgical unit (11) via connector (12). The electrical surgical unit (11) provides power and various controls. Figure 7B shows an exemplary device/assembly of the present disclosure, showing a detection component (20) attached to the medical device of Figure 7A. The detection component (20) is shown with two sense coils (30) inside housing (25). Also inside the house (25) is electronics component (35) which may, for example, be used to process the signals received by sense coils (30), and/or provide a display to a user regarding distance to a tag embedded in a patient.

Figure 6 shows an exemplary detection component (20) having a detection component housing (25) (e.g., composed of plastic or other material) which contains three sense coils (30) (which are arranged in a triangle configuration) and an electronics component (35). The housing (25) also has a device-securing opening (40) therein, which allows a medical device to be inserted and secured in place.

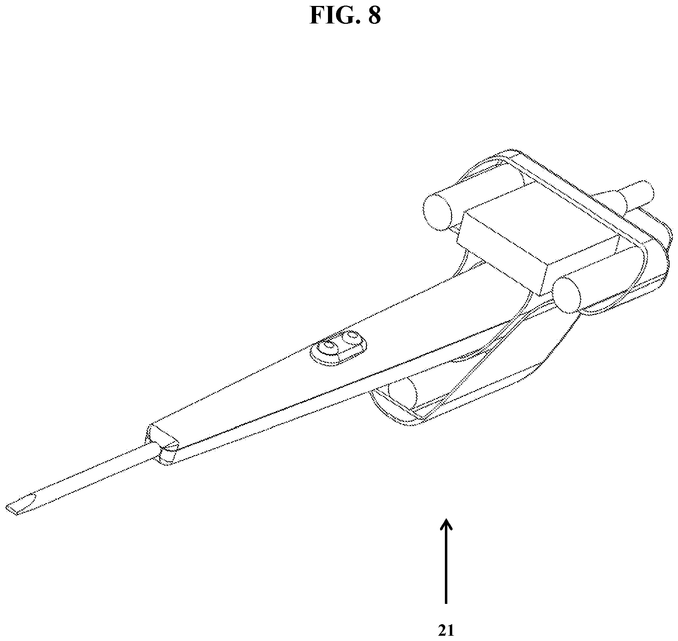

Figure 8 shows an exemplary detection component - medical device assembly (21), wherein the surgical device is inserted through the device-securing opening of the detection component housing. In this assembly, for example, the detection component is positioned such that it does not interfere with a user (e.g., surgeon) using the medical device in its normal mode of use. In this figure, the detection component is positioned distal to the cutting and/or cauterizing end of the medical device, and away from the buttons used during operation.

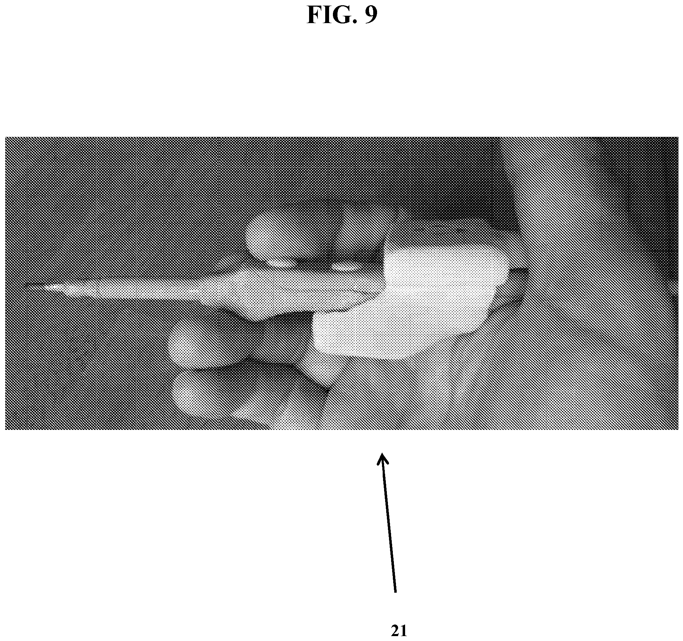

Figure 9 shown a photograph of an exemplary detection component-medical device assembly in the hand of a user. Again, the detection component is positioned such that the user is free to use the device and operate the buttons and cutting/cauterizing tip in a normal fashion.

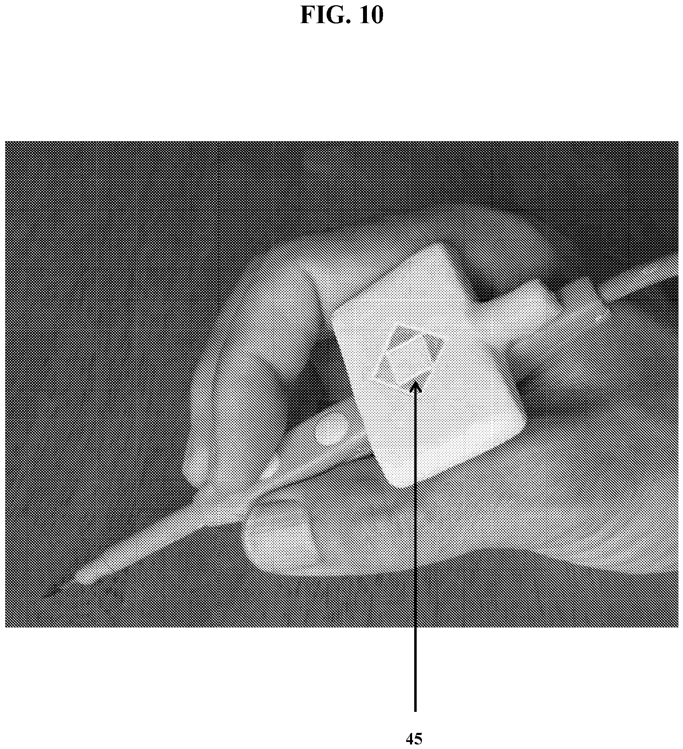

Figure 10 shows a photograph of an exemplary detection component with a visual display (45) located therein. A visual display may be used to inform the user (e.g., a surgeon) how far the tag (in the patient) is from the device, and may also be used to help keep the surgical device oriented in the correct planes (e.g., to avoid unnecessary cutting or cauterizing with the medical device). Orientation and/or distance may be indicated with a number of lights (e.g., 5 LED lights).

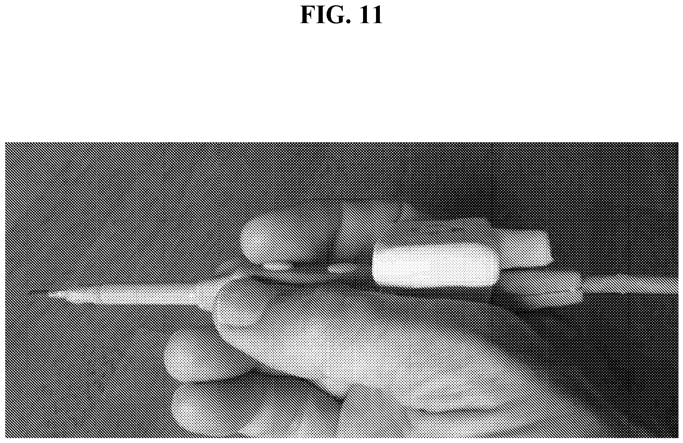

Figure 11 shows a photograph of a side view of an exemplary detection component-medical device assembly.

Figure 4 shows a patient with a tag (100) inserted next to a solid tumor (110) (e.g., in breast tissue of the patient), wherein the patient is laying on top of a remote activating device (250), which is shown as a flat pad. Also shown is a surgical device (200), which can be a detection component-surgical device assembly. The remote activating device may also be positioned closer to the tag (100) (e.g., by being placed on the abdomen), or placed further away (e.g., under the table or mattress the patient is supported on). The remote activating device (250) generates a magnetic field that passed through the patient's body, striking the tag, which causes a reflection or irregularity in the magnetic field. Such reflection or irregularity is detected by the detection component. A visual display (e.g., on the detection component or elsewhere) then reports the distance of the medical device tip (e.g., cutting and/or cautery tip) allowing the user (e.g., a surgeon) to precisely guide the medical device tip to the tumor. Prior to any cutting of tissue, the detection component - medical device may be moved all around the outside of the patient near the tag in order to calibrate the detection component.

Figure 5B shows a control unit (60) that is attached to both a remote activating device (50) and a detection component-medical device assembly (21). The remote activating device (50) has an excitation coil (55). The detection component - surgical device assembly (21) is attached to the control unit (60) via connection wire (65). When the power of the medical device is activated (e.g., to cut or cauterize) the control unit may turn off the magnetic field from the remote activating device, and then may turn the magnetic field back on when the power is not activated on the medical device. In this regard, any magnetic field generated by the medical device itself does not disturb the magnetic field generated by the remote activating device and vice versa. This help prevent the detection component from picking up false signals (from the medical device) that are not related to the location of the tag in the patient.

The technology is not limited by the mode of tag placement and a wide variety of placements techniques are contemplated (not being according to the claimed invention in view of Article 53(c) EPC) including, but not limited to, open surgery, laparoscopy, endoscopy, via endovascular catheter, etc. The tags may be placed by any suitable device, including, but not limited to, syringes, endoscopes, bronchoscopes, extended bronchoscopes, laparoscopes, thoracoscopes, etc. An exemplary protocol is provided below.

For surgical procedures, the patient may be placed onto an operating table with the surgical area exposed and sterilized. The surgeon is provided with the imaging information showing the location of the tumor and tag. An incision is made at the location of the entry point of the placement needle. The remote activating device is placed in proximity to the tissue to activate the tag. The detection component detects a signal from the tag and allow the surgeon to guide the direction medical device toward the tumor. Once the tumor is localized, the surgeon removes the appropriate tissue and removes the tag.

Use of this system and procedure significantly reduces procedure cost, time, and patient inconvenience as compared to wire placement and other unguided surgeries. Use of the tag reduces the number of imaging steps required and reduces time spent in radiology and surgery. Further, the patient is not left waiting for surgery with a wire hanging out of their body. Avoidance of the wire further reduces pain or discomfort associated with the pulling on the wire.

The detection component may be moved around the outside of the patient, sensing the tag at many different positions to build a 3D image of the location of tag within the tissue of the patient. Such data regarding the scan can, for example, be stored in the detection component or control unit and then used during a surgical procedure to determine the optimal point of entry into the patient's tissue, as well as the angle or angles which are best suited to approach the tag, and ultimately the associated tumor (e.g., to minimize cutting of non-target tissue and to maximize the removal of the tumor or tumors associated with the tag). Such 3D image scanning (e.g., prior to surgery) helps achieve the best result for the patient and helps reduce the need for repeating the procedure (e.g., to come back for parts of the tumor that were missed on the initial surgery).

The 3D image generated by moving the detection component around the location of a tag in a patient may be combined with an another image of the patient with the tag (e.g., generated by MRI, CT, etc.) to generate an image fusion. Combining two or more images of a patient using fiducials as marker points has been described previously (e.g., see, U.S. Pat. 7,848,553, and U.S. Pat. Pub. 20030153850). Commercial image fusion systems include STEALTHSTATION system and PATHFINDER system. Generating image fusions using a detection component described herein (e.g., where at least one or two implanted tags are used as fiducial points of reference), and then using the detection device for a procedure on a patient with the tags still in place, allows for real-time location correction for any movement of the patient (e.g., via breathing, changed position, organ movement during a packing procedure, etc.). In this regard, the detection components herein, and their guidance system (e.g., audible, tactile, or visuals signals) may be corrected during a surgical procedure (e.g., in real-time) so the operator is guided appropriately based on any changes in the patient tissue position (e.g., position of a tumor). The tag or tags serve as the fiducial points of reference for both the 3D image generated by the detection component, as well as the secondary image (e.g., from an MRI or CT image). The tags also then serve as fiducial points of reference during the procedure to orient the detection device and account for changes in position of the patient. The fiducials may be implanted in a subject (e.g., in breast tissue) or be external (e.g., such as placed on each earlobe prior to brain scans and subsequent brain surgery using the detection component and corresponding surgical device). To see the position, the tag may be used in combination with one or more other fiducials. For example, one tag in the breast and a sticker containing a fiducial on each shoulder. This type of real-time use of image fusion and location information may be used in any type of suitable surgical or ablative procedure, including for example, neurosurgery, hepatobiliary surgery, gynecological surgery, ENT surgery, urological surgery, etc.

Images (e.g., MRI, CT, etc.) that are generated for use with the detection component (and corresponding surgical device) may be marked to indicate the location of a target tumor, including a surgical margin around the tumor to ensure complete removal. A predetermined margin such as 0.5 ... 1 cm ... 1.5 cm ... 2 cm etc., may be set around a tumor to ensure removal. The surgical margin around a tumor could be set as a sphere, or drawn to correspond to any irregular shape of the tumor (e.g., hand drawn by a doctor on an image to match any irregular shape). This surgical margin around a tumor may be used such that, prior to using the detection component device and corresponding surgical device, one could calibrate for the signal intensity in the x, y and z axis related to this surgical margin such that whenever the device reaches a user defined boundary (the predetermined distance from the tumor) something changes, such as an audible, visual, or tactile signal (e.g., a yellow light when a user is at the surgical margin around tumor, and red light when the surgical device has gone within the predetermined surgical margin). There could be a signal warning that the surgical device is too close to the surgical margin (e.g., 5 mm), such as a red warning signal.

The tag may be placed at or near the tip of the surgical instrument or device to track its location (e.g., whether fused with a medical image or not). For example, the tag could be placed on or near the tip of a nasogastric tube or bladder catheter to confirm the tip position from outside the patient. This may be used to improve safety of surgical procedures. Also the tag may be placed on or near the tip of a vascular catheter, and the position of the catheter fused to a medical image (CT or MRI) to give the location of the instrument in the human body. Likewise for any surgical instrument, catheter, endoscopic instrument, sensing device, biopsy needle, or anything else inserted into the human body where the tip location is important, a tag may be used near or at the tip of such devices. Such as for simple applications, these could be unfused and the location determined from outside the body by a reader, or in the case of complicated anatomy, the location could be superimposed on a calibrated image set.

The detector component may comprise one or more lasers that are directed onto (e.g. projected onto) the surgical/procedure field (e.g., internal tissue of a patient) as a guide to a the user (e.g., as a guide to the target tumor that is to be resected). Multiple lasers may be used (e.g., all the same color or providing different colors). Such laser projection onto the surgical/procedure field allows the user (e.g., physician) to be guided to the target (e.g., tumor) without the need to look away from the surgical or procedure field. The detection component may be attached to a curved partially reflective lens that is, for example, be flipped up for easy viewing of the laser lights on the surgical field. Such a lens reflects the guiding lights towards the operator regardless of the orientation of the physician's head and the instrument surface. Such lens may be used to decrease parallax, and improve the viewing angle for the physician.

The display for the detection component may not part of or attached to the detection component, and instead may be remote. For example, the display component may be part of a head mounted, such as Google GLASS or similar devices that present a display close to a user's eye or eyes. In this regard, there may be a wireless connection between the detection component and the display, such as a BLUETOOH connection.

To energize the implanted marker(s), a single large-diameter coil, in the remote activating device, can be located near the patient (e.g., beneath the patient), for example in the cushion or bedding that supports the patient on the operating table. This single exciter antenna will generally exhibit the strongest magnetic field in a direction normal to the table, and hence may not coincide with the axis of an implanted marker. To maximize the excitation of a marker with arbitrary location and orientation while limiting patient exposure to safe levels of magnetic field, it is desirable to have multiple solenoidal coils arranged in sub-arrays that are substantially orthogonal to each other (e.g., as shown in Figure 13). These coils can further be subdivided into several shorter coils sharing the same axis, as shown in Figure 12.

Exemplary subdivided coils are shown in Figure 12. As depicted in this figure, a current source 1000 is used to drive an alternating current through one or more solenoidal coils 1004, which can be switched among these coils by switch 1002. For simplicity, only two coils are shown attached to the switch, and these coils are shown as independent, but it should be understood that some coils could be wired in series and that other switch or multiplexing techniques (including differential phasing or amplitudes of current sources) could be used to drive the array in a manner desired so that the implanted marker may be excited by a combination of coils, providing magnetic field to the marker while limiting the overall patient exposure to safe levels as determined by appropriate standards (e.g. IEEE). The spatial separation of these coils can be adjusted to enable a coarse localization of the marker with a given level of precision;, while, in general, closer spacing would require more coils (but could help to locate the marker more precisely).

The exciter coils themselves may be wound with magnet wire in one or more layers around a suitable core material, such as PVC, Teflon, or other low-loss and durable dielectric. The core could be solid, hollow, or filled with a ferrite material, depending on the number of turns and value of inductance desired. These coils would be of substantially similar inductances to facilitate resonant impedance matching to one or more current sources or generators 1000. The use of additional capacitors may assist in making each coil resonant, which could broaden the range of acceptable inductance values for different coils in the array 1006.

As shown in Figure 13, a set of sub-arrays of coils is arranged on a patient bed or cushion 2000. Longitudinal coils 2002 are arranged to approximately align with each breast, while a flat coil 2004 could encircle the orthogonal arrangement of longitudinal coils 2002 and lateral coils 2006. For simplicity, these coils are not shown as subdivided. They may be composed of several co-linear coils, each of which is independently activated to energize the marker while limiting total magnetic field to safe exposure levels. The coils in Figure 13 may be embedded in cushion 2000, packaged in their own separate enclosure, or suspended underneath the patient bed or table if the table's material properties do not unduly influence the magnetic field from the coils.

## Claims

1. A system comprising:
a) a tag (100), said tag (100) comprising a radio-frequency identification (RFID) chip and an antenna, wherein said tag (100) emits a sideband at a defined frequency, upon activation by a magnetic field;
b) a remote activating device (50, 250) that generates a magnetic field within a region of the tag (100);
**characterized in that** said system further comprises
c) a plurality of witness stations (30), each said witness station (30) comprising an antenna configured to detect said sideband; and
d) a component (20) that is attachable to a medical device (200), wherein said component (20) comprises:
i) one or more medical device location emitters that generate signals upon exposure to the magnetic field generated by said remote activating device (50, 250), said signals detectable by the plurality of witness stations (30), and
ii) an indicator comprising a visual display (45) that presents distance to tag information, and spatial orientation, for said medical device (200) accomplished geometrically by a procedure comprising measuring the quasi-simultaneous power detected at multiple witness stations (30).

2. The system of claim 1, wherein said remote activating device (50, 250) comprises an excitation coil (55, 252).

3. The system of claim 1, wherein the remote activating device (50, 250) comprises multiple coils (1004) arranged in sub-arrays (1006) that are substantially parallel to each other.

4. The system of claim 1, wherein said remote activating device (50, 250) comprises a pad configured to be placed in proximity to a patient having said tag (100) embedded in said patient.

5. The system of claim 4, wherein said pad further comprises said plurality of witness stations (30).

6. The system of claim 1, wherein each witness (30) station comprises a plurality of antennas that each feed a receiver channel that is time-division multiplexed.

7. The system of claim 1, further comprising a computer system that receives information from said plurality of witness stations (30) and generates information about a position of said tag (100).

8. The system of claim 1, wherein said component (20) comprises a sleeve that slips over a portion of said medical device (200).

9. The system of claim 1, wherein said medical device (200) comprises an electrocautery device.

10. The system of claim 1, wherein said antenna from each of said plurality of witness stations (30) is further configured to detect a signal from said one or more medical device location emitters.

11. The system of claim 1, wherein the remote activating device (50, 250) comprises a pad, and wherein the pad is placed under the patient or under a bed the patient is on.

12. The system of claim 1, wherein said tag (100) is implantable in a patient.

13. The system of claim 1, wherein said plurality of witness stations (30) is 4-12 witness stations.

14. The system of claim 1, wherein each of said witness stations (30) comprises
A) a ferrite-loaded cylindrical coil antenna, and
B) one or more capacitors.

15. The system of claim 14, wherein said ferrite-loaded cylindrical coil antenna is tuned for resonance at a frequency of 100-200 kHz.

## Patentansprüche

1. System umfassend:
a) ein Tag (100), wobei das Tag (100) einen Radiofrequenz-Identifikations (RFID)-Chip und eine Antenne umfasst, wobei das Tag (100) bei Aktivierung durch ein Magnetfeld ein Seitenband mit einer definierten Frequenz aussendet;
b) eine Fernaktivierungsvorrichtung (50, 250), die ein Magnetfeld in einem Bereich des Tags (100) erzeugt;
**dadurch gekennzeichnet, dass** das System des Weiteren umfasst
c) eine Vielzahl von Messstationen (30), wobei jede Messstation (30) eine Antenne umfasst, die zum Erfassen des Seitenbandes ausgebildet ist; und
d) eine Komponente (20), die an einer medizinischen Vorrichtung (200) anbringbar ist, wobei die Komponente (20) umfasst:
i) einen oder mehrere Sender zur Lokalisierung der medizinischen Vorrichtung, die Signale erzeugen, wenn sie dem von der Fernaktivierungsvorrichtung (50, 250) erzeugten Magnetfeld ausgesetzt werden, wobei die Signale von der Vielzahl von Messstationen (30) erfasst werden können, und
ii) ein Indikator, der eine visuelle Anzeige (45) umfasst, die Informationen über die Entfernung zum Tag und die räumliche Ausrichtung der medizinischen Vorrichtung (200) darstellt, die geometrisch durch ein Verfahren erreicht wird, das die Messung der quasi-simultanen Leistung umfasst, die an mehreren Messstationen (30) erfasst wird.

2. System nach Anspruch 1, wobei die Fernaktivierungsvorrichtung (50, 250) eine Erregerspule (55, 252) umfasst.

3. System nach Anspruch 1, wobei die Fernaktivierungsvorrichtung (50, 250) mehrere Spulen (1004) umfasst, die in Untergruppen (1006) angeordnet sind, die im Wesentlichen parallel zueinander sind.

4. System nach Anspruch 1, wobei die Fernaktivierungsvorrichtung (50, 250) eine Unterlage umfasst, die so konfiguriert ist, dass sie in der Nähe eines Patienten mit dem eingebetteten Tag (100) platziert werden kann.

5. System nach Anspruch 4, wobei die Unterlage des Weiteren die Vielzahl von Messstationen (30) umfasst.

6. System nach Anspruch 1, wobei jede Messstation (30) eine Vielzahl von Antennen umfasst, die jeweils einen Empfängerkanal speisen, der im Zeitmultiplexverfahren arbeitet.

7. System nach Anspruch 1, des Weiteren umfassend ein Computersystem, das Informationen von der Vielzahl von Messstationen (30) empfängt und Informationen über eine Position des Tags (100) erzeugt.

8. System nach Anspruch 1, wobei die Komponente (20) eine Hülle umfasst, die über einen Teil der medizinischen Vorrichtung (200) gezogen wird.

9. System nach Anspruch 1, wobei die medizinische Vorrichtung (200) eine Elektrokauterisationsvorrichtung umfasst.

10. System nach Anspruch 1, wobei die Antenne von jeder der Vielzahl von Messstationen (30) ferner so ausgebildet ist, dass sie ein Signal von dem einen oder mehreren Sendern für den Standort der medizinischen Vorrichtung erfasst.

11. System nach Anspruch 1, wobei die Fernaktivierungsvorrichtung (50, 250) eine Unterlage umfasst, und wobei die Unterlage unter dem Patienten oder unter einem Bett, auf dem der Patient liegt, platziert wird.

12. System nach Anspruch 1, wobei der Tag (100) in einen Patienten implantierbar ist.

13. System nach Anspruch 1, wobei die Vielzahl von Messstationen (30) aus 4-12 Messstationen besteht.

14. System nach Anspruch 1, wobei jede der Messstationen (30)
A) eine mit Ferrit beladene zylindrische Spulenantenne und
B) einen oder mehrere Kondensatoren
umfasst.

15. System nach Anspruch 14, wobei die mit Ferrit beladene zylindrische Spulenantenne auf Resonanz bei einer Frequenz von 100-200 kHz abgestimmt ist.

## Revendications

1. Système comprenant :
a) une étiquette (100), ladite étiquette (100) comprenant une puce d'identification radiofréquence (RFID) et une antenne, dans lequel ladite étiquette (100) émet une bande latérale à une fréquence définie, suite à son activation par un champ magnétique ;
b) un dispositif d'activation à distance (50, 250) qui génère un champ magnétique à l'intérieur d'une région de l'étiquette (100) ;
**caractérisé en ce que** ledit système comprend en outre :
c) une pluralité de stations témoins (30), chaque dite station témoin (30) comprenant une antenne configurée pour détecter ladite bande latérale ; et
d) un composant (20) qui peut être lié à un dispositif médical (200), dans lequel ledit composant (20) comprend :
i) un ou plusieurs émetteurs de localisation de dispositif médical qui génèrent des signaux suite à leur exposition au champ magnétique généré par ledit dispositif d'activation à distance (50, 250), lesdits signaux pouvant être détectés par la pluralité de stations témoins (30) ; et
ii) un indicateur comprenant un affichage visuel (45) qui présente une information de distance jusqu'à l'étiquette, et une orientation spatiale, pour ledit dispositif médical (200), tel que réalisé géométriquement par une procédure comprenant la mesure de la puissance quasi simultanée détectée au niveau de multiples stations témoins (30).

2. Système selon la revendication 1, dans lequel ledit dispositif d'activation à distance (50, 250) comprend une bobine d'excitation (55, 252).

3. Système selon la revendication 1, dans lequel le dispositif d'activation à distance (50, 250) comprend de multiples bobines (1004) agencées en sous-réseaux (1006) qui sont sensiblement mutuellement parallèles.

4. Système selon la revendication 1, dans lequel ledit dispositif d'activation à distance (50, 250) comprend un coussinet configuré pour être placé à proximité d'un patient comportant ladite étiquette (100) intégrée chez ledit patient.

5. Système selon la revendication 4, dans lequel ledit coussinet comprend en outre ladite pluralité de stations témoins (30).

6. Système selon la revendication 1, dans lequel chaque station témoin (30) comprend une pluralité d'antennes dont chacune alimente un canal de récepteur qui est multiplexé par répartition temporelle.

7. Système selon la revendication 1, comprenant en outre un système informatique qui reçoit l'information en provenance de ladite pluralité de stations témoins (30) et qui génère une information concernant une position de ladite étiquette (100).

8. Système selon la revendication 1, dans lequel ledit composant (20) comprend une gaine qui coulisse au-dessus d'une partie dudit dispositif médical (200).

9. Système selon la revendication 1, dans lequel ledit dispositif médical (200) comprend un dispositif d'électrocautérisation.

10. Système selon la revendication 1, dans lequel ladite antenne de chacune de ladite pluralité de stations témoins (30) est en outre configurée pour détecter un signal en provenance desdits un ou plusieurs émetteurs de localisation de dispositif médical.

11. Système selon la revendication 1, dans lequel le dispositif d'activation à distance (50, 250) comprend un coussinet, et dans lequel le coussinet est placé sous le patient ou sous un lit sur lequel le patient repose.

12. Système selon la revendication 1, dans lequel ladite étiquette (100) peut être implantée chez un patient.

13. Système selon la revendication 1, dans lequel ladite pluralité de stations témoins (30) comprend de 4 à 12 stations témoins.

14. Système selon la revendication 1, dans lequel chacune desdites stations témoins (30) comprend :
A) une antenne à bobine cylindrique chargée en ferrite ; et
B) un ou plusieurs condensateurs.

15. Système selon la revendication 14, dans lequel ladite antenne à bobine cylindrique chargée en ferrite est accordée pour une résonance à une fréquence de 100-200 kHz.
